# EUROPEAN PATENT SPECIFICATION

(11) **EP 3 073 868 B1**
(45) Date of publication and mention of the grant of the patent: **27.12.2017**
(21) Application number: 14828518.2
(22) Date of filing: 25.11.2014
(51) Int. Cl.: A47C 21/00, A47C 31/00, A61M 21/00

(54) **POLIFUNCTIONAL BED**
MULTIFUNKTIONSBETT
LIT POLYFONCTIONNEL

(30) Priority: 26.11.2013 IT TO20130959
(43) Date of publication of application: 05.10.2016
(73) Proprietor: Innersee S.r.l., 10129 Torino (IT)
(72) Inventor: BIANCOFIORE, Roberto, 10129 Torino (IT)
(74) Representative: De Bonis, Paolo
(86) International application number: PCT/IB2014/066318
(87) International publication number: WO 2015/079381

(56) References cited:
- WO-A1-00/62850
- JP-A- 2008 237 727
- JP-A- 2011 255 008
- KR-B1- 100 795 923
- US-A1- 2009 177 327
- US-A1- 2010 005 588

## Description

### Field of the invention

The present invention relates to multi-functional beds, in particular of the type designed for use in wellness or fitness centres or in medical centres. An example of a polifunctional bed of known type can be found in US 2009/0177327 A1. Other prior art documents in the same field include, for example, WO 00/62850 A1, KR-A-10-0795923, JP-A-2008-237727, US 2010/0005588 A1, JP-A-2011-255008.

### Prior art

Numerous examples of multi-functional beds for use in wellness or fitness centres or medical centres are known to the art. These beds in general have a structure that consists of a base carrying a frame (on which the user is received), which is constituted by a number of segments articulated together, the relative position of which is variable so that the frame can assume different configurations.

Provided that the frame is devised and designed so that a user can lie thereon, the various configurations are generally associated to different postures of the user's body, corresponding to different treatments being administered.

However, in many cases switching of the configuration of the frame, for example to pass from a supine or prone position of the user to a position in which the user is in a variably sitting position (for example, with back raised, legs raised, or both), may not be sufficient for proper and complete administration of a given treatment. In particular, there are some treatments that envisage administration of active agents, acoustic stimuli, or else visual stimuli (or a combination of two or more of the aforesaid stimuli), which hence require use of additional equipment. Such equipment may comprise, for example, masks for aerosols or else acoustic diffusers (possibly envisaging, alternatively, use of headphones)
or else spectacles of a type similar to the ones used for virtual-reality simulations.

It is thus evident that in these circumstances, the administration of the treatment may not be altogether comfortable for the user in so far as the area of the bed where the user's head is received is densely populated with external devices that, albeit functional for administration of the treatment, can hinder and/or limit the movements of the user.

In the case of use in the medical field, for example for administering an aerosol treatment, the presence of an aerosol mask and of tubes for supply of the mask may lead to intolerance on the part of the patient on account of the evident limitation of the head movements, which may render treatment more difficult to tolerate.

The problem is, from certain standpoints, further amplified in the case of application in wellness centres or the like, where the requirements of comfort are at the very basis of administration of the treatment. Furthermore, there is undoubtedly a presence of external devices in a decidedly higher number given the greater number of stimuli generally envisaged by the treatment protocols. On the hypothesis of having external devices for diffusion of fragrances and for diffusion of binaural tunes or audio signals, which call for the use of headphones, as well as external devices for visual stimulation, it is evident that the result of the treatment may in effect prove very distant from the expectations of the user in terms of well-being.

### Object of the invention

The object of the invention is to provide a multi-functional bed on which it is possible to administer a treatment (both of stimulation of the body and stimulation of the mind) guaranteeing maximum freedom of movement both for the user and for the operator who is administering the treatment, as well as the maximum comfort possible for the user who receives the treatment.

### Summary of the invention

The object of the invention is achieved by a multi-functional bed having characteristics set out in claim 1. Preferred features of the invention are set out in dependent claims.

### Brief description of the drawings

The invention will now be described with reference to the annexed drawings, which are provided purely by way of non-limiting example and in which:
- Figure 1 is a perspective view of a multi-functional bed according to various preferred embodiments of the invention, where the bed is represented in a first configuration;
- Figure 2 corresponds to Figure 1 but represents the bed in a second configuration;
- Figure 3 is a cross-sectional view according to the trace III-III of Figure 2;
- Figure 4 is a view corresponding to that of Figure 3 but illustrating an example of optical stimulation;
- Figures 5 and 6 are perspective detailed views according, respectively, to the arrow V of Figure 1 and the arrow VI of Figure 2;
- Figure 6A is a cross-sectional view according to the trace VI-VI of Figure 5;
- Figure 7 is a partially exploded perspective view of a functional assembly indicated by the arrow VII in Figure 2;
- Figure 7A is a cross-sectional view according to the trace VII/A-VII/A of Figure 7, whilst Figure 7B is a cross-sectional view according to the trace VII/B-VII/B of Figure 7;
- Figures 8, 8A, 8B are similar to Figures 7, 7A, 7B but refer to a variant of the aforesaid functional assembly (the traces for the section are, however, VIII/A-VIII/A and VIII/B-VIII/B);
- Figure 8C is a cross-sectional view according to the trace VIII/C-VIII/C of Figure 8;
- Figure 9 is a view corresponding to that of Figure 6 but referring to administration of a treatment to a user;
- Figure 10 is a view according to the arrow IX of Figure 8;
- Figure 11 is a view corresponding to that of Figure 3 but illustrating a further preferred embodiment of the bed according to the invention;
- Figure 11A is an enlarged view of a detail indicated by the arrow XI_A in Figure 11, viewed from the opposite side of the bed;
- Figure 12 is a view of a portion of the bed of Figure 11 with a user lying thereon; and
- Figures 13 and 14 are, respectively, an exploded view and a cross-sectional view of a further embodiment of a diffusion module for the bed of Figure 11.

### Detailed description of preferred embodiments of the invention

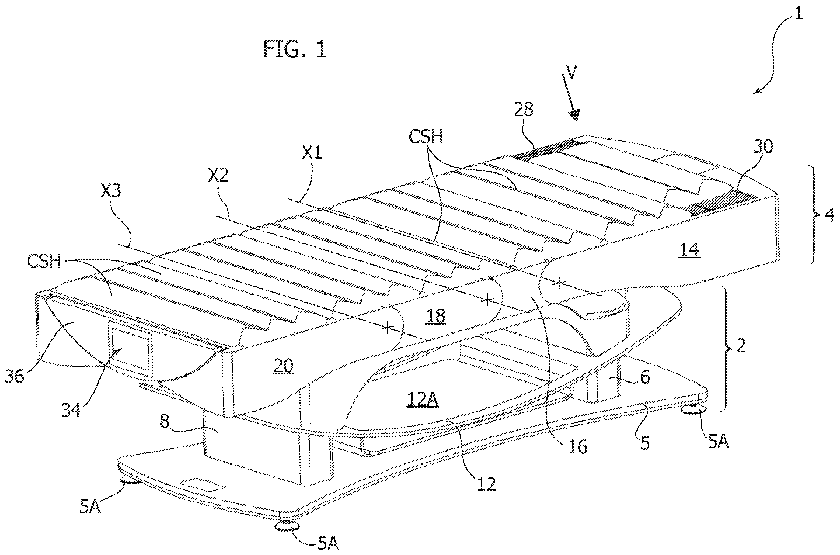

In Figure 1, the reference number 1 designates as a whole a multi-functional bed according to various preferred embodiments of the invention. The bed 1 includes a base 2 and a frame 4 supported by the base 2. With reference to Figures 1 to 3, the base 2 includes a base plate 5 provided with stabilization feet 5A configured for resting on the floor and on which a first telescopic upright 6 and a second telescopic upright 8 are installed. The telescopic uprights 6, 8 are moreover connected to a further plate 10, which supports the frame 4.

A shelf structure 12 extends in a bridge-like fashion between the telescopic uprights 6 and 8 and includes a shelf 12A useful for accommodating objects such as towels, cosmetic articles, medical products or various equipment.

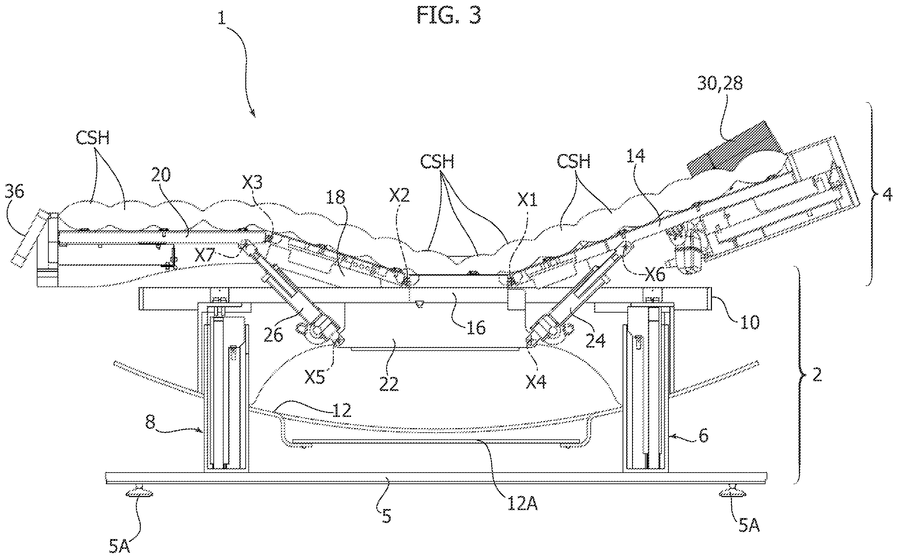

The telescopic uprights 6, 8 are operable between a retracted position, illustrated in Figure 3, and an extracted position (not illustrated) so as to vary the height of the supporting plate 10, and consequently of the frame 4, from the floor.

The frame 4 includes two or more segments articulated together, the relative position of which is variable to obtain different configurations of the frame 4 itself. In the embodiment here considered preferential, the frame 4 includes four articulated segments designated by the reference numbers 14, 16, 18, 20. Of these, the segment 16 is fixed and fastened to the plate 10, whereas, as regards the other segments:
- the segment 14 is articulated to the segment 16 about a transverse axis X1;
- the segment 18 is articulated to the segment 16 about a transverse axis X2 parallel to the axis X1; and
- the segment 20 is articulated to the segment 18 about a transverse axis X3 parallel to the axis X2.

Movement of the segments 14, 18, 20 is entrusted to a drive unit including a framework 22 fixed underneath the plate 10 in an area comprised between the telescopic uprights 6 and 8, and a first linear actuator 24 and a second linear actuator 26.

The framework 22 carries a first hinge element and a second hinge element that define respective hinge axes X4, X5, which also have a transverse orientation and are moreover parallel to one another and parallel to the axes X1-X3. The aforesaid hinge elements function as connection point for corresponding hinge elements provided on respective first ends of the linear actuators 24 and 26. Preferentially, these actuators are of an electromechanical type with screw/nut mechanism. The second ends of the actuators 24 and 26 are instead hinged, respectively, to the segment 14 and to the segment 20 about hinge axes X6, X7, which are also transverse and parallel to one another and to all the axes previously mentioned.

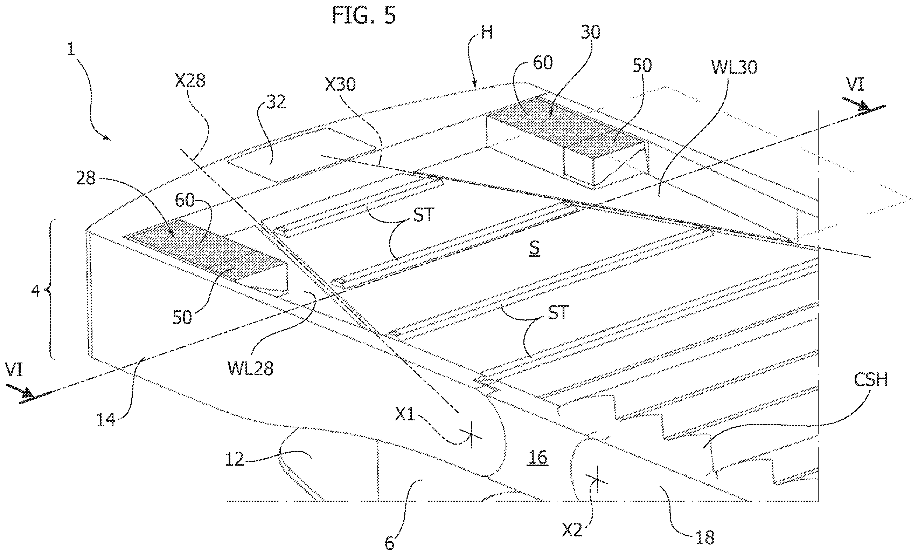

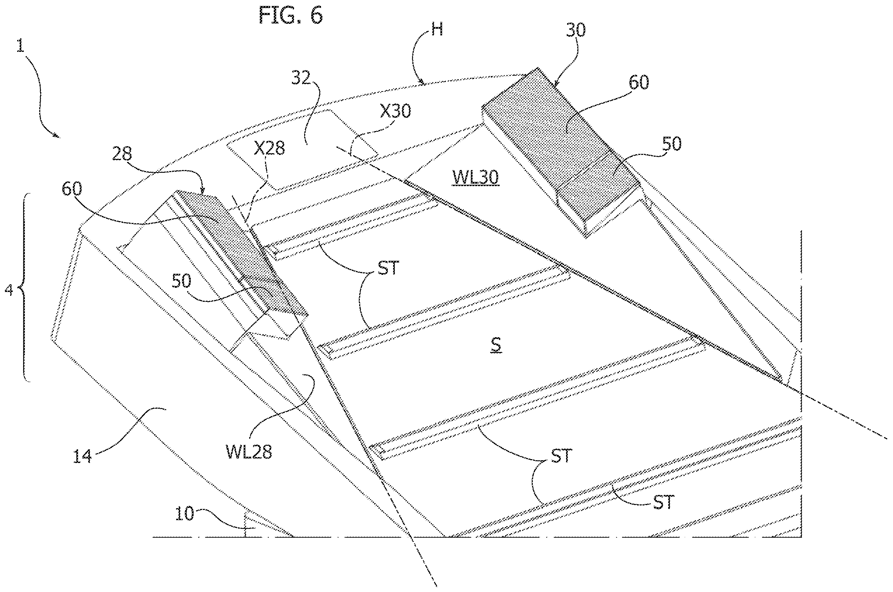

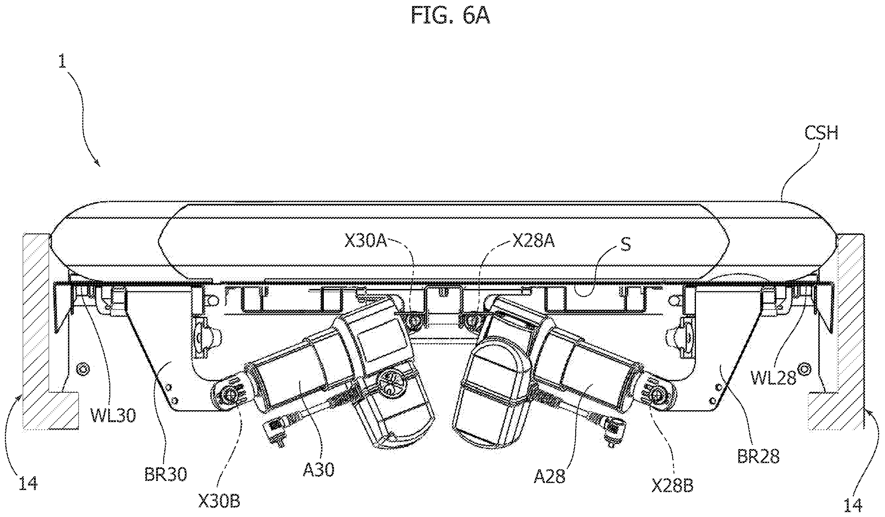

With reference in particular to Figures 5 and 6, arranged within the perimeter of the frame 4 defined by the succession of the segments 14 to 20 are plates S that form a surface for supporting the user's body. This surface preferentially comprises a sequence of plates S equal in number to the segments 14-20 and fastened thereto. In this way, each plate S follows the movements of the segment 14-20 to which it is coupled, giving rise to various configurations of the surface that receives the user's body.

On the plates S there may moreover be installed a plurality of devices, comprising, amongst other things (in combination or alternatively, according to the requirements):
- a plurality of strips ST including a plurality of light sources, in particular polychrome LED light sources or light sources of an RGB or RGBW type;
- a plurality of pad-type electrical-resistance heaters that function as heating elements for one or more padded cushions CSH lying on the sequence of plates S for supporting the user/patient; and/or
- a plurality of tactile transducers, preferably of a vibrational type, configured for tactile stimulation of a user/patient.

The cushions CSH may be padded with a liquid or a gel permeable to visible light (so that the light radiation emitted by the sources ST traverses the cushions CSH and is exploited by the user) or with sponge-rubber or other foamed material (for example, a shape-memory material), possibly coated with leather.

In the latter case, the strips ST are not strictly necessary in so far as the padding could not guarantee permeability to light (or in any case the coating could not so allow). However, it may be convenient in any case to install the strips ST in so far as the cushions CSH are in themselves an interchangeable component.

To go into in greater detail as regards the structure of the frame 4, the segment 14 includes an end region, designated as a whole by the reference H and corresponding also to an end region of the frame 4. The end region H is configured for receiving the head of a user who is lying on the bed 1.

Installed in the end region H, on opposite sides in a transverse direction, are a first diffusion module 28 and a second diffusion module 30, which are identical from the functional standpoint but are structurally specular since - as will be seen in what follows - their arrangement is such that they must be located on opposite sides with respect to the head of a user lying on the bed 1.

The first and second diffusion modules 28, 30 are mobile between a resting position and a working position. According to the invention each diffusion module 28, 30 is rotatable about a respective axis of rotation X28, X30, where the axes X28 and X30 are coplanar and convergent. In order to enable this oscillation, each diffusion module 28, 30 is installed on a respective mobile winglet WL28, WL30, of course oscillating about the respective axis X28, X30. The amplitude of the rotation of the wings WL28 and WL30 about the axes X28 and X30 that can be set ranges between 0° and 90°.

The wings WL28 and WL30 preferentially have a right-triangular shape with hypotenuse parallel to the axis X28 or X30 (respectively) and incorporate the diffusion modules 28, 30 in a point corresponding to the right angle between the legs of the triangle.

The wings WL28 and WL30 are driven in rotation via electromechanical actuators (visible in Figure 6A), which are preferentially once again of the external screw/internal screw type with lever system to enable correct range of oscillation of the wings. In particular, with reference to Figure 6A, associated to each of the wings 28, 30 is a linear electromechanical actuator designated by the reference number A28, A30m respectively. A first end of the actuators A28, A30 is hinged to the plate S in a position corresponding to the segment 14 about an axis X28A, X30A, respectively, whereas a second end of the actuators A28, A30 (preferentially, the end of a stem thereof) is hinged to a corresponding connection bracket BR28, BR30 about an axis X28B, X30B fixed to the corresponding wings WL 28.

Further details as regards the structure of the end region H, in particular of the diffusion modules 28, 30 will be provided in what follows.

Further integrated in the segment 14, once again in the end region H, is a device for projection of images, which is designated as a whole by the reference number 32, in itself known, and consists of a projector the light beam of which impinges on one or more mirrors for projection of static images or films that are to be displayed by the user, for example via a screen SC set above the bed 1.

Provided at the opposite end of the frame 4, in particular on the segment 20, is a control panel 34 operatively connected to an electronic control unit (not visible) configured for driving all the actuators on board the bed 1, the projection device 32, and the diffusion modules 28, 30. In the preferred embodiment represented in the figures the control panel is designated by the reference number 34 and comprises a touch screen installed on a swinging panel 36, which can turn about a transverse axis parallel to the axes X1-X7. The control panel 34 thus performs the function of human-machine interface.

The structure of the diffusion modules 28, 30 will now be described in detail, in particular as regards the preferred embodiment represented in the figures.

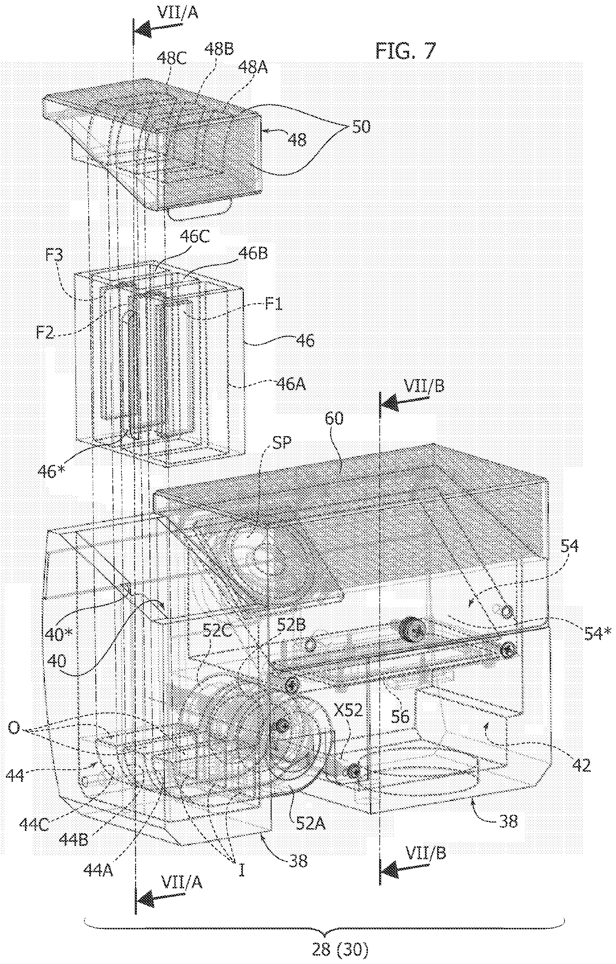

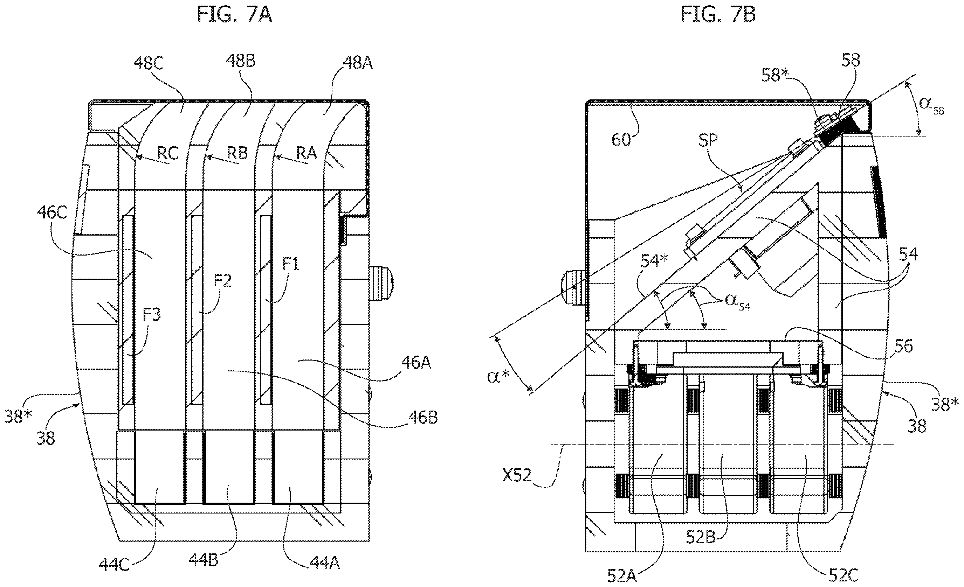

With reference to Figure 7, in a first preferred embodiment, each diffusion module 28, 30 (here illustrated specifically is the module 28 but - as the reference 30 appearing in brackets indicates - the description may apply perfectly also to the other diffusion module) comprises a base body 38 including a first cavity 40 and a second cavity 42. The base body 38 further includes a dorsal surface 38* having a shape convex outwards in order to prevent any objects from accidentally falling in the gap that separates the module 28, 30 from the perimeter of the segment 14 during rotation of the modules 28, 30; otherwise, in the case where the wall 38* were plane, this gap would remain exposed. Furthermore, the conformation of the surface 38* minimises the risk of an operator/physician who is administering the treatment, or else of the user/patient, getting his or her fingers pinched.

The first and second cavities 40, 42 may be provided within a single box-shaped body that defines the base body 38 or else they may be provided on independent box-shaped bodies (as in the preferred embodiment illustrated herein), which are joined together to form the base body 38.

The cavity 40 has a generally parallelepipedal shape, which is closed on the bottom and gives out at the top onto an inclined surface and functions as housing for a device for diffusing active agents. By the term "active agent" is meant any substance that is able to interact with the human body, even, for example, with simple stimulation of a receptor. In this way, included in the category are substances with therapeutic properties in the strict sense (for example, pharmaceuticals) and substances provided with therapeutic properties in a broad sense (basically of a non-pharmacological type, for example perfumes or flavours for wellness treatments) or without therapeutic properties.

In general, the basic structure of a device for diffusion of active agents for use in the diffusion modules 28, 30 is of the type including one or more channels, each of which communicates with a corresponding fan configured for sending a flow of air therein, and housed in each channel is a cartridge containing the active agent.

In the preferred embodiment illustrated herein, the diffuser of active agents is a fragrance diffuser built as described in what follows.

Housed inside the cavity 40 is a first cartridge element 44, in which three flow ducts 44A, 44B, 44C are provided. Each flow duct 44A, 44B, 44C includes an inlet section I and an outlet section O located on mutually orthogonal surfaces, between which walls with generally curvilinear geometry develop.

Also inserted into the cavity 40 is a second cartridge element 46 having a parallelepipedal shape, which carries within it three flow ducts 46A, 46B, 46C having the same section of passage as that of the outlet sections O of the ducts 44A-44C and configured as a prolongation of the ducts 44A-44C themselves, as on the other hand may be noted from the dashed-and-dotted lines that represent installation of the cartridge 46 within the cavity 40.

When inserted in the cavity 40, the cartridge element 46 bears upon the element 44 and preferentially, is guided via a projection 47 that slidably engages a groove 40* provided in the wall of the cavity 40. Inserted inside each duct 46A, 46B, 46C is a cartridge F1, F2, F3 (respectively) containing one or more fragrances/flavours. It should be recalled, however, that it is possible to use the cartridges F1-F3 charged with other active agents, for example medical products. Each cartridge F1, F2, F3 is removably fixed within the corresponding duct 46A, 46B, 46C, for example within a wall niche (see, in this connection, the cross-sectional view of Figure 7A).

The cartridges F1-F3 may moreover be charged with one and the same fragrance/flavour (or in general active agent) or with fragrances/flavours (or in general active agents) that may differ according to the requirements. The advantages will emerge in any case more clearly in what follows.

The cartridge elements 46, 44 are packed tight together in the cavity 40 via a lid 48 which also bears curved-wall ducts designated by the reference numbers 48A, 48B, 48C. These ducts constitute, respectively, the prolongation of the sequence of the ducts 44A and 46A, 44B and 46B, 44C and 46C, as represented once again by the dashed-and-dotted lines. The lid 48 is moreover preferentially coated at the top with a perforated layer 50 that masks the openings of the ducts 48A-48C on the outside and is at the same time permeable to air. There are in any case possible embodiments in which the perforated layer 50 is absent: in this case, there remains only the lid 48 with the openings of the ducts 48A-C exposed to the air.

Three centrifugal fans 52A, 52B, 52C are fixed to the base body 38 in such a way that the respective delivery mouths are in view of and connected to the inlet sections I of the channels 44A, 44B, 44C, respectively. Preferentially, the centrifugal fans 52A-52C are installed in an area comprised between the cavities 40 and 42.

The above fans are of a known type and preferentially include an axial intake port located in a plane orthogonal to an axis of rotation X52 of the respective impellers and a tangential delivery port that is joined to (and has the same area of passage as) the inlet section I of a corresponding duct 44A, 44B, 44C.

With reference to Figures 7 and 7A, set within the cavity 42 is a V-shaped plate, designated by the reference number 54. The V-shaped plate 54 is installed so that one of the two flaps of the V shape is set parallel to and in contact with the walls of the cavity 42, whilst the other flap is positioned so as to offer on the outside an inclined surface 54*, on which an acoustic diffuser SP is mounted, for example a common diffuser of a magnetodynamic type.

The groove defined by the plate 54 is moreover closed at the bottom via a passive radiator 56, which improves the sound yield of the diffuser SP in the low-frequency range.

At the vertex of the base body 38, at the interface with the V-shaped plate 54, a strip 58 is provided including a plurality of light sources, preferentially, of a polychrome LED type. Moreover set at the top of the ensemble constituted by the acoustic actuator SP and by the strip 58 is a lid 60, with a structure similar to that of the lid 48 (i.e., preferentially provided with a coating having an openwork structure permeable to air or, alternatively, without any coating with openwork structure, with consequent exposure of the openings of the ducts 48A'-C' to the air) and set flush therewith.

Some characteristic geometrical parameters of the diffusion modules 28, 30 will now be described in detail.

As regards the ducts 48A-48C, the radius of curvature of the walls, designated by R in Figure 7A, is comprised in the range 5-85 mm, preferentially, 28-50 mm, more preferentially, 33-45 mm. The V-shaped plate 54, and in particular the surface 54* on which the acoustic diffuser SP is installed, is inclined with respect to the horizontal by an angle α₅₄ comprised between 0° and 45°.

Finally, a plane 58* of the strip 58, installed on which are the LED light sources (or likewise light sources of an RGB type, or other sources) is inclined with respect to the horizontal by an angle α₅₈ comprised between 0° and 90°.

Preferentially, the angles α₅₄ and α₅₈ are not chosen identical to one another since, as will be described more fully in what follows, they have to be optimised according to the position of the receptors that are able to capture the stimuli sent by them, namely eyes and ears.

For this reason, it is possible to indicate also an angular range within which an angle of inclination between the two surfaces, 54* and 58*, falls where the light sources of the strip 58 are located. The amplitude of the angle between the two surfaces, designated by α* in Figure 7B, is defined by the difference α₅₄ - α₅₈ and is generally comprised in the range 5°-20°, preferably 8°.

Also the choice of the radii of curvature R of the walls of the ducts 48A-48C is made in such a way that the flow of air that exits therefrom impinges in the most targeted way possible upon the user's nose, as will be seen more fully in what follows. In this connection, in the preferred embodiment illustrated herein, the ducts 48A, 48B, 48C each have a respective radius of curvature R_{A}, R_{B}, R_{C} chosen in the range 20-60 mm, with R_{A} < R_{B} < R_{C}, and chosen preferentially, with the following values: R_{A} = 28 mm, R_{B} = 33 mm, R_{C} = 45 mm.

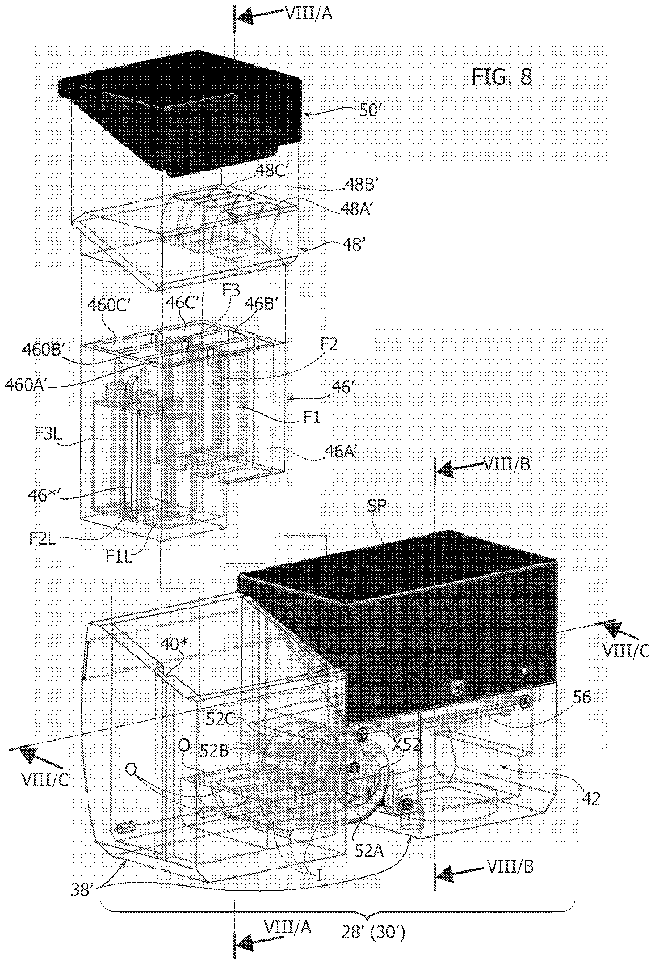

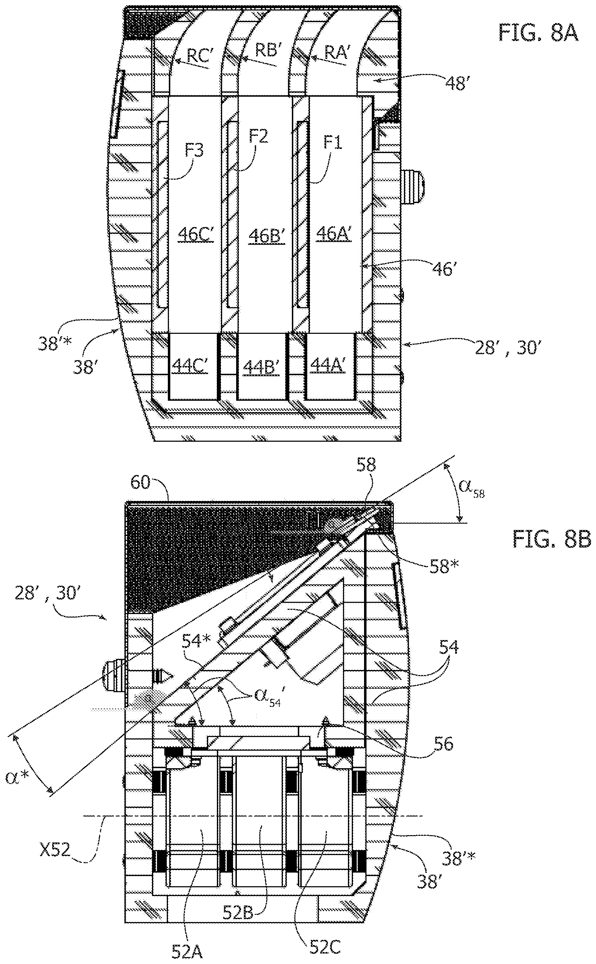

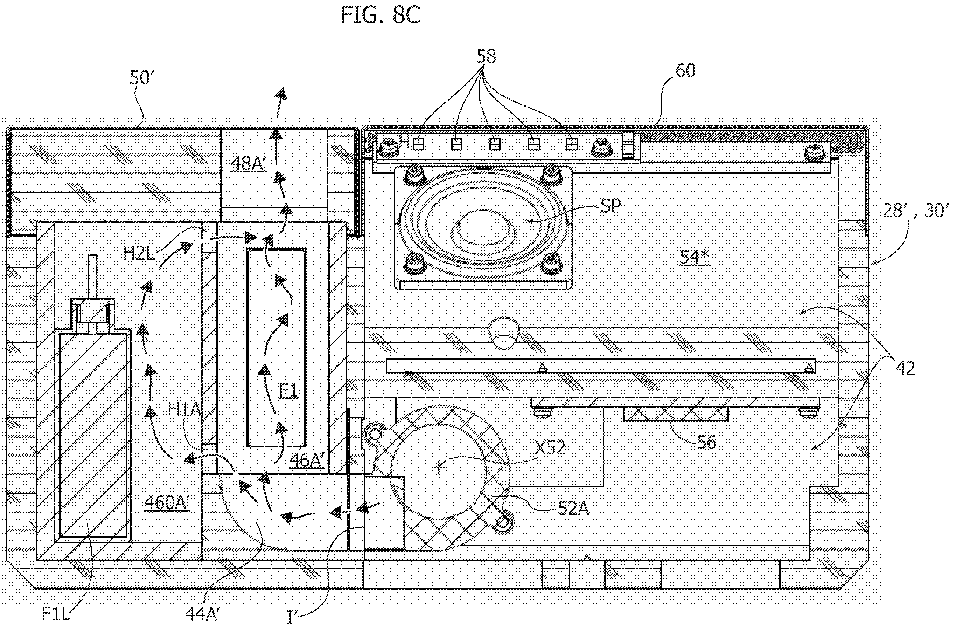

With reference to Figure 8, a second preferred embodiment of the diffusion modules will now be described, designated now by the references 28', 30' (specifically illustrated here is the module 28' but - as the reference 30' in brackets indicates - the description applies in the very same way also to the other diffusion module). All the components identical or homologous to the ones described previously are designated by the same reference numbers. The diffusion module 28' comprises a base body 38' including a first cavity 40' and the second cavity 42.

The first and second cavities 40', 42 may be obtained within a single box-shaped body that defines the base body 38' or else may be obtained on independent box-shaped bodies, as in the preferred embodiment illustrated herein, which are joined together to form the base body 38'. The base body 38' moreover includes a dorsal surface 38*' having a shape convex outwards in order to prevent any objects accidentally falling in the gap that separates the module 28', 30' from the perimeter of the segment 14 during rotation of the modules 28', 30'; otherwise, in the case where the wall 38*' were plane, this gap would be exposed.

The cavity 40' has a generally parallelepipedal shape closed on the bottom and giving out at the top onto an inclined surface and functions as housing for a device for diffusing active agents. The basic structure of the device for diffusing active agents for use in the diffusion modules 28', 30' is - like in the case of the modules 28, 30 - of the type including one or more channels, each of which communicates with a corresponding fan configured for sending a flow of air therein. However, as will be seen hereinafter, in this embodiment each channel is of doubled in order to enable housing of a cartridge containing an active agent in the solid state and a cartridge containing an active agent in the liquid state, and in which moreover the two portions of each channel are in communication with one another to enable mixing of the two types of active agent.

In the preferred embodiment illustrated here, the diffuser of active agent is a fragrance diffuser made as described in what follows.

Housed within the cavity 40' is a first cartridge element 44' in which three flow ducts 44A', 44B', 44C' are provided. Each flow duct 44A', 44B', 44C' includes an inlet section I and an outlet section O located on mutually orthogonal surfaces, between which there develop walls with generally curvilinear geometry.

Moreover inserted in the cavity 40' is a second cartridge element 46' having a parallelepipedal shape, which carries within it three flow ducts, each dividing into two parallel ducts 46A' and 460A', 46B' and 460B', 46C' and 460C'. The ducts 46A'-46C' have the same section of passage as the outlet sections O of the ducts 44A'-44C' and are configured as prolongation of the ducts 44A-44C themselves. The ducts 460A'-460C' are separated from one another but communicate with the ducts 44A'-44C', respectively, through a first port H1A and a second port H2A.

When inserted in the cavity 40, the cartridge element 46 bears upon the element 44 and is preferentially guided via a projection 47 that slidably engages a groove 40*' provided in the wall of the cavity 40'. Inserted within each duct 46A', 46B', 46C' is the cartridge F1, F2, F3 (respectively) containing one or more fragrances in the solid state. It should be recalled, however, that it is possible to use cartridges F1-F3 charged with other active agents, for example medical products. Each cartridge F1, F2, F3 is removably fixed within the corresponding duct 46A', 46B', 46C', for example within a wall niche (see, in this connection, the cross-sectional view of Figure 8A) .

The cartridges F1-F3 may moreover be charged with one and the same fragrance/flavour (or in general active agent) or with different fragrances/flavours (or in general active agents) according to the requirements.

Housed, instead, in the ducts 460A'-460C' are three vials of fragrance F1L, F2L, F3L in the liquid state.

The cartridge elements 46', 44' are packed tight together in the cavity 40' via a lid 48', which also bears curved-wall ducts designated by the reference numbers 48A', 48B', 48C'. These ducts constitute, respectively, the prolongation of the sequence of the ducts 44A' and 46A', 44B' and 46B', 44C' and 46C'. As regards the geometry of the ducts 48, and in particular the values of radius of curvature thereof, in this case the same values already provided as regards the ducts 48A-48C apply. In particular, the radius of curvature is comprised in the range 5-85 mm, preferably 28-50 mm, more preferably 33-45 mm.

Furthermore, in the preferred embodiment illustrated herein, the ducts 48A', 48B', 48C' each have a respective radius of curvature R_{A}', R_{B}', R_{C}' chosen in the range 20-60 mm, with R_{A}' < R_{B}' < R_{C}', and chosen preferentially, with the following values: R_{A}' = 28 mm, R_{B}' = 33 mm, R_{C}' = 45 mm.

The lid 48' is moreover coated at the top with a perforated layer 50' that masks the openings of the ducts 48A'-48C' on the outside and is at the same time permeable to air. It is to be noted that the ducts 460A'-460C' do not communicate directly with the ducts 44-46-48 (A-C), but by way of the holes H1A, H2A (as will emerge clearly in what follows).

Each of the modules 28', 30' moreover includes the three centrifugal fans 52A, 52B, 52C fixed to the base body 38 in such a way that the respective delivery mouths are in view of and connected to the inlet sections I of the channels 44A', 44B', 44C', respectively. Preferentially, the centrifugal fans 52A-52C are installed in an area comprised between the cavities 40' and 42.

The structure of the cavity 42, including the components arranged within it, does not vary with respect to the modules 28, 30: the description of the cavity 42 already made previously applies altogether to the modules 28', 30'. Also the modalities of installation of the modules 28', 30' on the wings WL28 and WL 30, as likewise their operation (resting and working positions, angle of rotation about the axes X28 and X30) is identical to what has already been described and what will be described hereinafter for the modules 28, 30.

Operation of the bed 1 is described in what follows.

The bed 1 is of a multi-functional type in so far as it is configured both for administration of treatments with essentially mechanical action, for example physiotherapy massage or relaxing massage of various nature, and for administration of treatments with therapeutic action, for example of an active agent (for instance, aerosol or else, in the non-medical field, perfumes and flavours) and/or of visual or acoustic stimuli.

The extreme flexibility of the bed 1 is guaranteed by the presence of the diffusion modules 28, 30, which are able to carry out an extremely large number of the functions normally performed by external devices usable in combination with a bed of a known type. The fact that they are of a movable type moreover enables improvement of ergonomics and effectiveness of treatment, and guarantees for the operator who carries out the treatment maximum flexibility of operation.

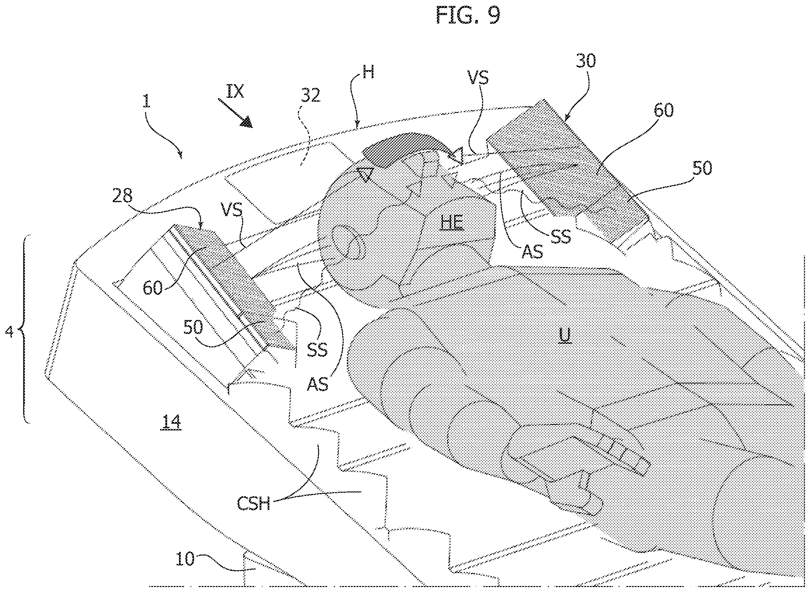

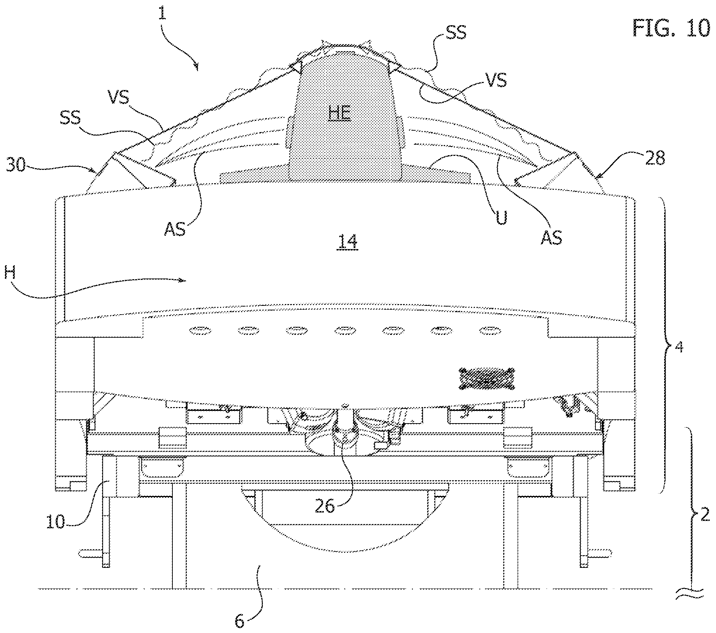

Whatever the treatment administered, a user U (Figures 9 and 10) must lie down on the bed 1 so that his or her head, designated by the reference HE, is positioned in the end region H. In this way, in view of the position of the wings WL28 and WL30 and of the diffusion modules 28, 30 on opposite sides of the end region H, the user's head HE will be received between the diffusion modules 28, 30 and the wings WL28 and WL30.

The diffusion modules are movable between a resting position and a working position by oscillating about the axes X28, X30.

In the resting position, the wings WL28, WL30 are located substantially flush with the plate S coupled to the segment 14, whilst the diffusion modules 28, 30 are set substantially flush with the frame 4, i.e., with the perimetral elements of the segment 14. For greater clarity, the resting position of the wings WL28 and WL30 and of the diffusion modules 28, 30 is illustrated in Figure 5.

It is to be noted that in general the resting position of the diffusion modules 28, 30 is associated to an aligned configuration of the segments 14, 16, 18, 20 that make up the frame 4, which is functional for the user to assume a prone or supine position for administration of a massage treatment.

With this position of the wings WL28, W130 and of the diffusion modules 28, 30 the working area - in the end region H - for the operator who administers the treatment to the user is extremely rational and clean. The fact that the aforementioned components are positioned flush with the adjacent structures guarantees the absence of sharp edges and other potential sources of impact, both for the user and for the operator.

In the working position, the wings WL28, WL30 and the diffusion modules 28, 30 are moved about the axes X28, X30 so as to arrange them oriented in the direction of the head HE of the user U, who is lying on the bed 1.

The aforesaid working position is illustrated in Figures 6, 9, and 10.

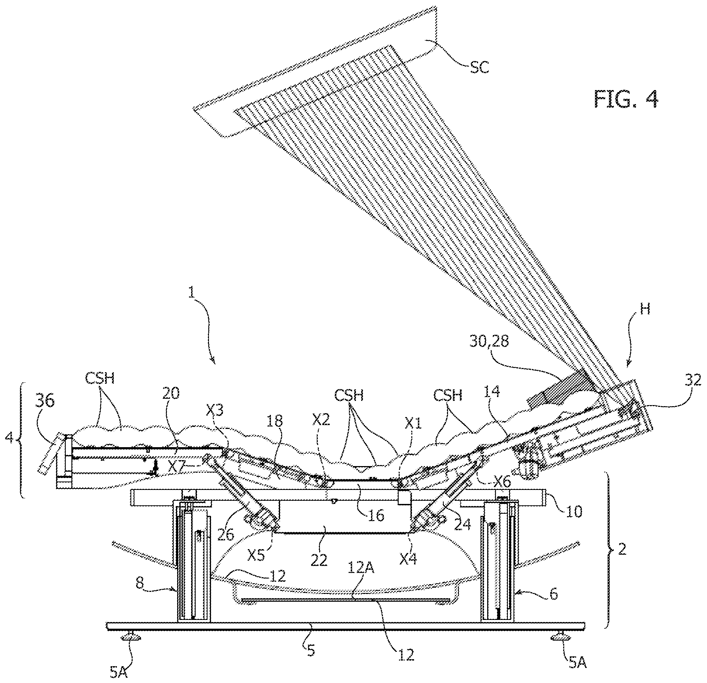

It will be appreciated on the other hand that in general the aforesaid working position is associated to a configuration with staggered levels of the frame 4 (see, for example, Figures 3 and 4), which can be obtained by operating the actuators 24, 26 via the control panel 34 or else by control at a distance (wire-control, remote-control, pedal-driven control). This configuration is functional for reaching a position where the user is more or less sitting and is characterized by the inclination of the segment 14 and the segment 18 with respect to the segment 16, whilst the segment 20 remains substantially in a horizontal position. Preferentially, the inclination with respect to the horizontal of the segment 14, associated to which is the back of the user U, is in general variable in the range 0°-45° (where the value 0° corresponds to the configuration of Figure 1).

Of course, the configuration appearing by way of example in Figures 3 and 4 may be varied at will by acting on the actuators 24, 26.

With the aid of Figures 4, 9, and 10, the modalities of administration of a treatment with therapeutic action of a non-pharmacological type will now be described. The description that follows is developed with reference to a treatment that can be administered by means of the modules 28, 30 in the configuration described herein (and represented in Figures 7, 7A, 7B), which enables them to provide olfactory, visual, and acoustic stimuli, but of course administration of the three types of stimuli may be altogether independent, and embodiments may be envisaged in which equipment of the modules 28, 30 is more limited so that they are suited to administering treatments characterized by a smaller number of stimuli. In the treatment here described, there is preferentially envisaged, in combination, a further stimulation using the projection device 32, the tactile transducers installed on the plates S, and, in the case where the bed is equipped with the cushions CSH, also the strips of light sources ST.

It should moreover be recalled that in totally different fields of application, for example the hospital field, instead of the stimulations listed above, there may be envisaged, as main stimulation, the auditory one: in the case, for example, of patients in coma, it is possible to diffuse through the diffusers SP the recorded voice of relatives, family, or other loved ones so as to increase the possibility of exit from the comatose state.

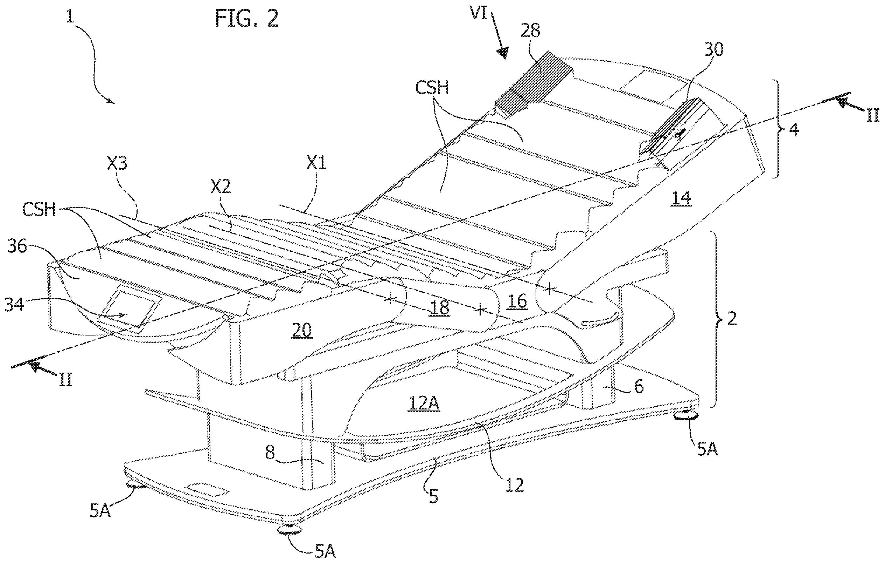

The user U lies down on the bed 1, which may have the frame 4 indifferently in the configuration of Figure 1 or in the configuration of Figure 2 so that his or her head HE is positioned in the end region H, between the wings WL28 and WL30 and between the modules 28, 30.

The user U moreover rests on the cushions CSH, which can be heat conditioned via the pad-type electrical-resistance heaters arranged on the plates S; in this way, heating is produced to a variable extent so as to guarantee always the best level of comfort possible for the user.

The strips of light sources ST installed on the plates S can moreover create luminous effects aimed at generating a particular sensation and/or a particular emotive involvement in the user, provided that the materials of the cushions CSH and the padding (water or gel) are chosen with characteristics of permeability to visible light.

By bringing the diffusion modules 28, 30 into the working position (Figures 9 and 10) it is possible to start a topical stimulation, in particular involving the head HE of the user U. Movement of the diffusion modules is managed through the control panel 34, which communicates with the electronic control unit of the bed 1 that controls all the actuators thereof, in particular the ones responsible for movement of the modules 28, 30. Alternatively, it is possible to exploit a control at a distance (wire-control, remote-control, pedal-driven control).

The electronic control unit that governs the bed 1 includes a plurality of treatment/therapy protocols stored therein and selectable through the control panel 34. To these treatment/therapy protocols there correspond different combinations of visual, acoustic, and olfactory stimulations. In practice, this results in as many combinations of commands issued to:
i) centrifugal fans 52A-C, responsible for olfactory stimulation. The fans 52A-C, send air into the sequence of ducts 46 (A, B, C)-48 (A, B, C), thereby impinging upon the cartridges F1, F2, F3 so as to bring into suspension in the fluid current the particles of flavour/perfume trapped in the cartridges F1-F3 and diffuse them towards the user's nose through the ducts 48. Each centrifugal fan 52A-C can be driven in a completely independent way for the purpose of regulating the intensity of the olfactory stimulation and of creating mixtures of fragrances in which (possibly) one or other flavour is render prevalent, this basically being obtained by varying the speed of rotation and the flow processed by the fans;
ii) strips of light sources 58 and ST, responsible for part of the visual stimulation; the colour, intensity, and possible intermittence of the light sources may be varied electronically at will;
iii) acoustic diffusers SP, responsible for acoustic stimulation; in particular, it is possible to select the frequencies and sounds to be reproduced, regulate the volume of reproduction and equalization of the sound; the audio traces may be conveniently stored in the control unit of the bed 1 or else on a removable mass memory interfaceable with the control unit itself.

With reference to point i) above, in the case where the bed 1 is equipped with the diffusion modules 28', 30', it is possible to have available yet a further degree of freedom in the olfactory stimulation of the user who receives the treatment. In particular, with reference to Figure 8C, the flow of air delivered by the centrifugal fans 52A-C within the sequence of ducts 44'-46'-48' also flows within the ducts 460A'-C' (through the holes H1A) which contain the vials F1L-F3L. The flow of the air into the ducts 460A'-C' impinges upon the mouth of the vials and creates a negative pressure that sucks the liquid fragrance outwards, nebulizing it (in a way at least roughly resembling the principle of operation of a carburettor for an internal-combustion engine); the fluid current with nebulized droplets of fragrance in suspension exits from the ducts 460A'-C' through the holes H2L and proceeds (see the path in Figure 8C) towards the ducts 48A'-C', aggregating with the flow of air that carries in suspension solid particles of fragrance eroded from the cartridges F1-F3.

In this way it is possible not only to add a further three fragrances to the mixture (the total is now six fragrances), but it is also possible to mix fragrances in the solid state and in the liquid state, exploiting the respective advantages thereof.

As regards the visual stimulation, the treatment protocols stored in the control unit also comprise corresponding instructions for management of the projection device 32, which forms an integral part of the system of visual stimulation of the bed 1.

More in general, when a specific treatment protocol from among the ones available is activated, initialization and management of different activities is obtained, amongst which:
i) activation and movement of the servo mechanisms that govern the mobile parts of the bed 1, including the servo mechanisms that act on the wings WL28, WL30; in this way, the wings are kept flush with the segment 14 when the operator/physician is acting on the body of the user/patient, and are then displaced into the working position when the bed 1 enters into a completely "sensorial" operating mode; the above is obtained in predefined times and with ranges of displacement and speeds that are also predefined;
ii) interaction between the fragrances and/or the dosage thereof and/or their mixing where this is required;
iii) adjustment of the audio volume, audio frequencies, tactile vibrations, both in terms of amplitude and in terms of frequency and phase (complete modulation);
iv) turning-on and turning-off of the device for projection of images 32 and evocation of visual and auditory scenarios selected in the mass memory on board the bed; powering of the projection device 32 is moreover preferably performed in such a way as to minimize energy consumption;
v) RGB or RGBW light stimulation (using light sources ST, as well as, possibly, light sources 58), with modulation in frequency, tone and shade of colour, amplitude, and phase; the tone and shade of the RGB or RGBW bands may be varied either on a single colour or on a number of colours sequentially (wave effect);
vi) adjustment of the temperature of the cushions CSH; it is to be noted in any case that this can also be done manually by the operator according to the needs.

The operations referred to above are carried out in an altogether autonomous way and are initialized and performed according to the type of protocol chosen by the operator/physician. As has been mentioned, and with reference to Figures 9 and 10, the choice of the angles α₅₄ and α₅₈, the radii R of the ducts 48A-48C and, not least, the angle of rotation about the axes X28 and X30 necessary for bringing the modules 28, 30 into the working position (preferentially comprised between 0° and 90°) is made so as to guarantee that the olfactory stimuli (designated by SS), the visual stimuli (designated by VS), and the acoustic stimuli (designated by AS) impinge in the most precise and targeted way possible on the corresponding receptor, namely nose, eyes, ears.

In other words, each diffusion module 28, 30 (or 28', 30') includes a number of "active portions" (i.e., the portions that are provided with the various stimulation devices - the fragrance diffuser, the acoustic diffuser SP, and the light sources of the strip 58), which have an orientation such that, when the module 28, 30 (or 28', 30') is in the working position, the stimulus emitted by them impinges in a targeted way upon a corresponding receptor of the human body.

It will be appreciated then how the bed 1 according to the invention enables significant results to be achieved in terms of quality of the treatment since it is possible to administer all the stimulations without any hindrance by wires, masks, or other external devices that may surround the user's head and that may possibly pass over of the patient's body, as instead occurs with beds of a known type.

In the bed 1 according to the invention, the majority (if not all) of the sources of stimulus are enclosed in the diffusion modules 28, 30, which are positioned laterally with respect to the head (and the body) of the user without hindering the latter's movements and without limiting them.

Of course, the bed 1 may be adapted to specific requirements simply by varying the equipment thereof, and in particular the diffusion modules 28, 30 provided. In general, in the bed 1 according to the invention the diffusion modules 28, 30 may be configured for diffusion of at least one from among an active agent, a sound signal, and a visual signal.

For this reason, embodiments may be envisaged in which the modules 28, 30 include a single source of stimulation amongst the ones described previously. For example, in the case where the bed 1 is used for administration of treatments via inhalation route, just the system of ducts and centrifugal fans described previously may be sufficient. In this case, instead of the fragrances, the cartridges F1, F2, F3 will be charged with a pharmacologically active agent (for example, a composition for aerosol), which would reach in a precise and controlled way the airways of the patient thanks to the choice of the geometrical parameters according to the modalities described.

For such applications it is possible to provide a single centrifugal fan and cartridge elements 44, 46 (as well as the plug 48) with a single duct, since generally the active agent to be administered is just one. However, if there were the need to administer a mixture of pharmaceuticals via inhalation route, this could be obtained with the same modalities with which the mixtures of flavours described previously are administered.

Also the presence of the projection device 32 may in itself be optional, in the case in point in the case where this is not required by the specific treatment that is to be administered with the aid of the multi-functional bed 1.

Irrespective of the set of instruments provided, the considerable ergonomics of the entire system both for the person receiving the treatment and for an operator/physician who has to administer the treatment to a patient/user remain unvaried, this deriving from the extremely clean and rational structure of the bed 1, in both of positions (the resting position and the working position) of the modules 28, 30 (and 28', 30'). Operation of the modules 28, 30 (and 28', 30') may moreover be completely automated according to the modalities with which the operator/physician decides to intervene on the user/patient. In this connection, specific operating protocols may be envisaged just the modules 28, 30 (and 28', 30').

Of course, the details of construction and the embodiments may vary widely with respect to what has been described and illustrated herein purely by way of example, without thereby departing from the scope of the present invention. It will thus be appreciated that the embodiments described herein are to be understood as being provided by way of non-limiting example, and particular embodiments are possible that include combinations of parts different from the ones illustrated herein and combinations of components that do not include all the components illustrated herein.

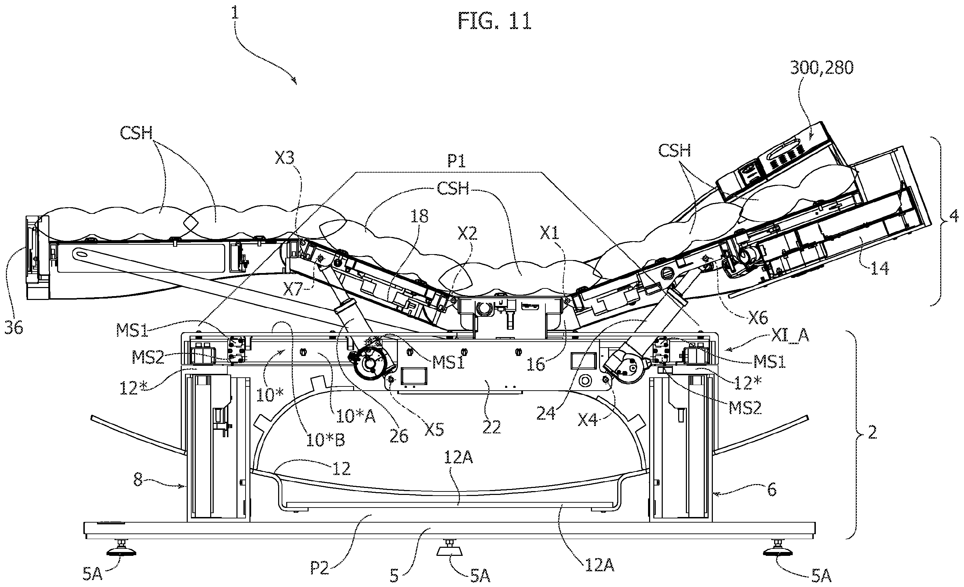

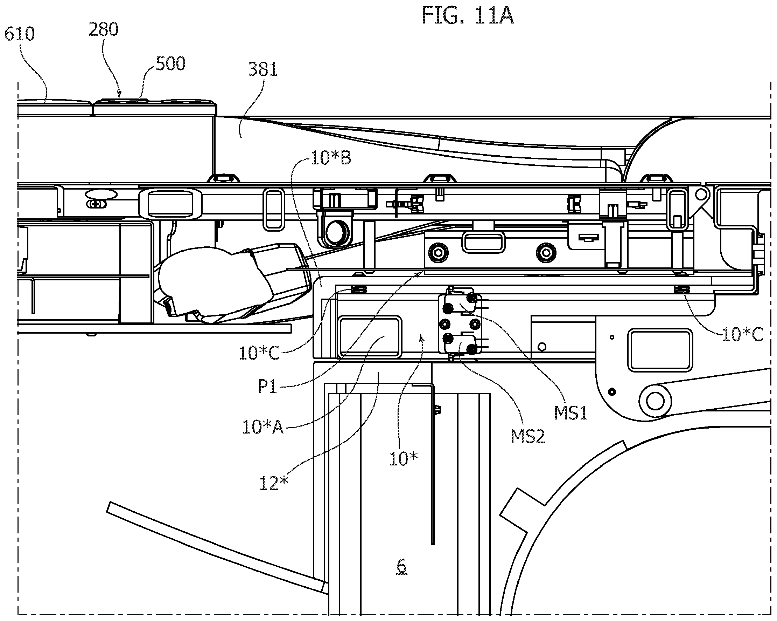

With reference to Figure 11, in a further preferred embodiment of the bed 1 a plurality of anti-pinch devices is provided to prevent the possibility of damage caused by the mobile parts of the bed both to the operator who administers the treatment and to the patient/user, as well as to objects forming part of the equipment.

In general, the bed 1 includes in this embodiment a plurality of anti-pinch devices configured for stopping movement of one or more mobile parts of the bed (for example, the shelf 12A, the segments 14, 18, 20, and the diffusion modules in any embodiment thereof) with respect to one or more elements of the bed that remain fixed with respect to the above one or more mobile parts during the aforesaid movement, where a command for arrest of the movement is issued when a foreign body enters the path of the movement itself.

A person skilled in the branch will in fact appreciate that on the bed 1 there exist numerous areas involved in the movement of the mobile parts of the bed that may constitute a source of risk for the operator who administers the treatment, the patient, or both.

A first of these areas is designated by P1 in Figures 11 and 11A and corresponds to the area subtended by the frame 4, in particular when the segments 14, 18, and 20 are raised. A first anti-pinch device (MS1) is configured for stopping the movement of one or more of the segments 14, 18, 20 in the case where a foreign body comes to be present on a supporting element of the frame 4 in a position subtended by the segments 14, 18, 20.

In order to install the aforesaid anti-pinch device, the plate 10 is preferentially replaced by a double supporting element 10* constituting the supporting element of the frame 4 with respect to which the action of the first anti-pinch device is effective. The double supporting element 10* includes a supporting frame 10*A, and a cover 10*B, which shields the frame 10*A and is mounted elastically with respect thereto by means of a plurality of elastic supports 10*C (here, by way of example, pins with annexed helical spring fitted thereon).

Fixed to the frame 10*A (Figure 11A) is a plurality of safety micro-switches MS1 that are configured for being activated when the cover 10*B approaches the plate 10* caused, for example, by the fact that one or more objects are rested on the cover 10*B or else the operator or the patient have rested their hands or arms thereon while the segments of the frame 4 are being lowered towards the cover 10*B. In such an eventuality, in the case where the force applied on the cover 10*B by the arm or hand were not in itself sufficient for activation of the micro-switches MS1, as soon as the segments of the frame 4 move into a condition where they are about to pinch the arm or hand, the force transmitted to the latter will in any case be greater than the threshold necessary for activation of the micro-switches MS1. The threshold of activation chiefly depends upon the elastic pre-loading of the elastic supports 10*C.

In the preferred embodiment of Figures 11-11A, the supporting element 10* includes six safety micro-switches MS1, four of which are arranged in the proximity of the four corners of the supporting element 10*, and the last two are arranged in a position corresponding to the actuator 26 but on opposite sides of the frame 10*A.

A second anti-pinch device is instead configured for stopping the movement of the telescopic uprights 6, 8 of the base 2, which support the frame 4, in the case where a foreign body comes into contact with the shelf structure 12 - suspended between the telescopic uprights 6, 8 and vertically mobile therewith - during a movement of descent thereof.

In particular, the second anti-pinch device is effective in regard to pinching of a foot or hand (or other foreign body, including an object) underneath the shelf structure 12, in particular between the shelf 12A and the base plate 5.

The device again exploits a plurality of safety micro-switches, here designated by the reference MS2, which are fixed to the frame 10*A and are configured for being activated by means of flat horizontal end plates 12* of the shelf structure 12. In greater detail, the shelf structure 12 is supported in a floating way by the telescopic uprights 8, and in particular is without vertical constraint in the direction of raising of the shelf structure 12 with respect to the base plate 5.

As a result of this, in the case where a foot or hand (or an object) were to remain trapped between the shelf structure 12 and the base plate 5 during a descending movement of the telescopic uprights 6, 8, since the plate 5 is fixed, exchange of forces deriving from the pinching action would cause lifting of the shelf structure 12 and a consequent activation of the micro-switches MS2. Preferentially, four micro-switches MS2 are provided substantially at the four corners of the frame 10*B (like the corresponding micro-switches MS1) .

Activation of each micro-switch MS1, MS2 (which may also be independent in the case where pinching were to produce an unbalancing of the cover 10*A or of the shelf structure 12) causes an immediate interruption of power supply:
- in the case of the micro-switches MS1, to the actuators 24 or 26 or both; it should be noted in this connection that the cover 10*B is divided into two portions, with a first portion associated to the head of the bed 1, and a second portion associated to the feet of the bed 1, whereby there is possible an independent action of the micro-switches MS1 located at opposite ends of the frame 10*B; and
- in the case of the micro-switches MS2, to the actuators that govern the telescopic uprights 6, 8.

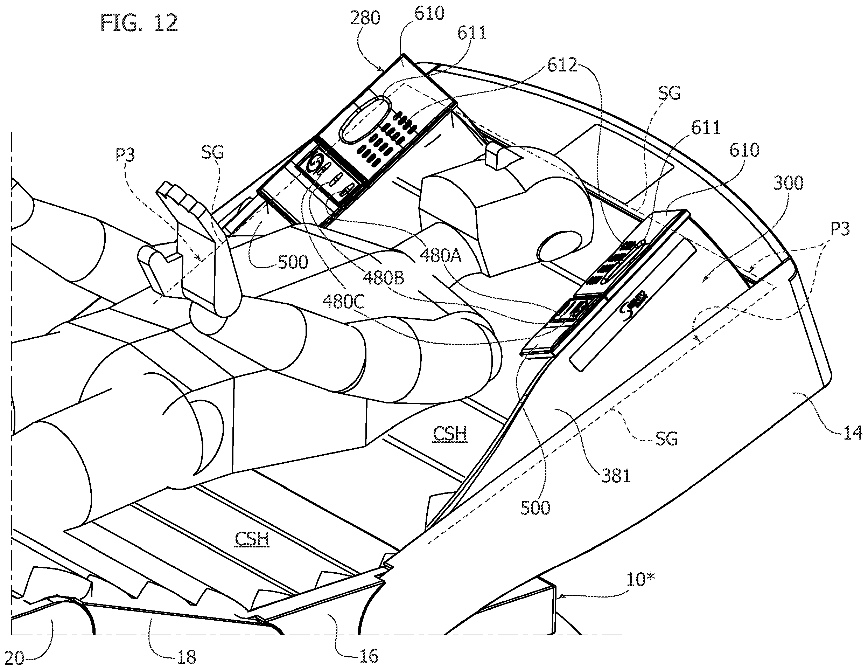

With reference to Figure 12, the bed 1 moreover includes a third anti-pinch device (SG) configured for stopping the rotary movement about the axes X28, X30 of the first diffusion module and/or of the second diffusion module in the case where a foreign body comes to be pinched between them and the segment 14 that receives the head of a user.

The third anti-pinch device exploits a plurality of sensitive edges SG arranged along the perimeter of the segment 14 and in a position corresponding to the gap between the latter and the diffusion modules, which constitutes a third area of pinching identified by the reference P3.

It should in any case be noted how in general there may be envisaged embodiments in which the bed 1 is equipped with just one or only some of the anti-pinch devices mentioned.

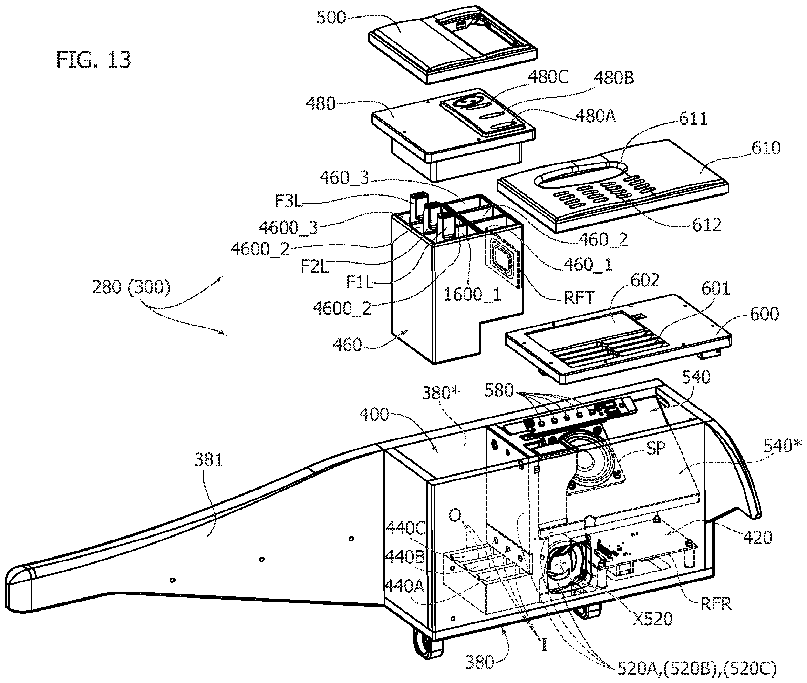

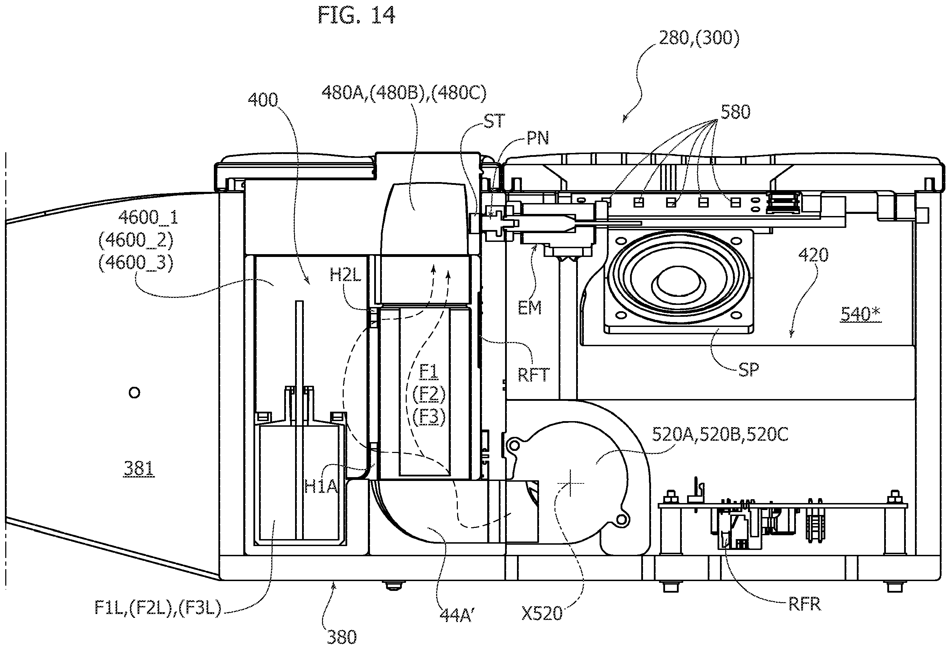

In this embodiment, as will emerge more clearly from the description of the subsequent Figures 13 and 14, the diffusion modules have undergone further modifications that are in part synergistic for preventing pinching and in part aimed at increasing the possibilities in the administration of treatments. For this reason, the diffusion modules of Figures 11 to 14 are designated by the references 280, 300.

The diffusion modules 280, 300 are identical to one another from the functional standpoint but are structurally specular in so far as they are located on opposite sides with respect to the head of a user lying on the bed 1.

Like the modules 28, 30, also the modules 280, 300 are mobile between a resting position substantially flush with the frame 4 and a working position (Figure 13); each diffusion module 280, 300 is for the purpose rotatable about the axis of rotation X28, X30, respectively, and is installed on the mobile wings WL28 (module 280) and WL30 (module 300), naturally oscillating about the axes X28, X30, respectively.

The ensuing description will be developed for just the module 280, but it also applies of course to the module 300.

With reference to Figures 13 and 14, the diffusion module 280 comprises a base body 380 including a first cavity 400 and the second cavity 42.

The first and second cavities 400, 42 are provided within a single box-shaped body that defines the base body 380.

The base body 380 moreover includes a dorsal surface 380*, which, in addition to having a convex shape like the corresponding dorsal surfaces of the diffusion modules 28, 30, 28', 30' already described, is prolonged by means of a wing-shaped portion 381 which is also convex towards the outside in order to shield further the gap that separates the module 280 from the perimeter of the segment 14 during rotation of the module itself.

The cavity 40' has a generally parallelepipedal shape closed on the bottom and functions as housing for a device for diffusing active agents. The basic structure of the device for diffusing active agents is - like in case of the modules 28, 30, 28', 30' - of the type that includes one or more channels, each of which communicates with a corresponding fan configured for sending a flow of air therein. Each channel is doubled to enable housing of a cartridge containing an active agent in the solid state and a cartridge containing an active agent in the liquid state, and where moreover the two portions of each channel are in communication with one another to enable mixing of the two types of active agents.

In the preferred embodiment here illustrated, the diffuser of active agent is a fragrance diffuser obtained in the way described in what follows.

With reference in particular to Figures 13 and 14, provided inside the cavity 400 are three flow ducts 440A, 440B, 440C, each including an inlet section I and an outlet section O located on mutually orthogonal surfaces, between which there develop walls having a generally curvilinear geometry.

Inserted in the cavity 400 is a cartridge element 460 having a substantially parallelepipedal shape, which carries inside it three flow ducts, each dividing into two parallel ducts 460_1 and 4600_1, 460_2 and 4600_2, 460_3 and 4600_3. The ducts 460_1, 460_2, 460_3 have the same section of passage as the outlet sections O of the ducts 4600_1, 4600_2, 4600_3 and are configured as a prolongation of the ducts 440A-440C themselves. The ducts 4600_1-4600_3 are separated from one another but communicate with the ducts (respectively) 460_1-460_3 through a first ?port H1A and a second port H2A.

Inserted within each duct 460_1, 460_2, 460_3 is the cartridge F1, F2, F3 (respectively) containing one or more fragrances in the solid state. It should be recalled, however, that it is possible to use cartridges F1-F3 charged with other active agents, for example medical products. Each cartridge F1, F2, F3 is removably fixed within the corresponding duct 460_1, 460_2, 460_3, for example within a wall niche.

The cartridges F1-F3 may moreover be charged with one and the same fragrance/flavour (or in general active agent) or with different fragrances/flavours (or in general active agents) according to the requirements.

Housed, instead, inside the ducts 4600_1-4600_3 are three vials of fragrance F1L, F2L, F3L in the liquid state.

In order to identify the type and/or combination of fragrances contained in the cartridges F1-F3 or F1L-F3L, the cartridge element 460 is provided with a transponder RFID designated by the reference RFT. The transponder RFT is configured to communicate the information that it contains to a reader RFR, which is configured for querying the transponder RFT and interacting with the control unit of the bed 1, which is programmed for controlling the reader RFR whenever information contained in the transponder RFT is required.

The radio-frequency transponder RFT in general contains information corresponding to at least one of the following:
- a code identifying the origin of the cartridge element 460; this enables, for example, unique identification of the original cartridge elements, and for this purpose the transponder RFT is queried whenever insertion of a new and/or different cartridge element 460 is detected;
- one or more active agents (F1, F2, F3, F1L, F2L, F3L) contained in the aforesaid cartridge element (460); in this way, it is possible to make available the information regarding the type and composition of the active agents to the control unit of the bed 1, which can act on the operating parameters thereof accordingly and can moreover signal any possible incompatibility between the treatment to be administered to the patient/user and the characteristics of the active agents contained in the cartridge element 460; and
- an initial optimal setting of operating parameters of the bed 1 according to characteristics of the one or more active agents contained in the cartridge element 460.

Nevertheless, it is possible to use the transponder RFT for generation of status messages regarding the residual amount of consumables (for example, the level of liquid in the vials F1L-F3L). For this purpose, the transponder RFT can be combined to one or more level sensors of a known type, for example optical or ultrasound sensors.

The cartridge element 460 is closed in the cavity 400 via a lid 480, which also has curved-wall ducts designated by the reference numbers 480A, 480B, 480C.

The lid 480 is moreover coated at the top with a gel cushion 500.

Three centrifugal fans 520A, 520B, 520C (these are not visible in the figures and are thus indicated in brackets: as regards their arrangement see, for example, Figure 7 or Figure 8) are fixed to the base body 380 in such a way that the respective delivery mouths are in view of, and connected to, the inlet sections I of the channels 440A, 440B, 440C, respectively.

The structure of the cavity 420, including the components arranged therein, do not differ from that of the modules 28, 30. The description of the cavity 42 already developed previously applies altogether to the modules 280, 300. In particular, set within the cavity 420 is a V-shaped plate designated by the reference number 540. The V-shaped plate 540 is installed so that one of the two flaps of the V-shaped plate is set parallel to and in contact with the walls of the cavity 420, whereas the other flap is positioned so as to offer on the outside an inclined surface 540*, on which an acoustic diffuser SP is mounted, for example a common diffuser of a magnetodynamic type.

At the top of the base body 38, at the interface with the V-shaped plate 54, a strip 580 is provided including a plurality of light sources, preferentially of a polychrome LED type.

Furthermore, in this embodiment housed within the cavity 420 is an electromagnet EM with an anchor connected to a blocking pin PN that is received in a seat ST. When the electromagnet is energized, the blocking pin AN is pushed into the seat ST, preventing (accidental or intentional) extraction of the lid 480 and access to the cartridges of active agent.

Set at the top of the cavity 420 is a lid 600 including a grid 601 for sending out the audio signal of the diffuser SP, and a window made of opalescent material for improving diffusion of light of the light sources of the strip 580.

Likewise fixed to the lid 600 is a gel cushion 610, which is open in an area corresponding to an oval window 611 superimposed on the window 602 and to a plurality of openings 612 superimposed on the grill 601.

In combination with the action of shielding afforded by the wing-shaped portion 381, the modules 280, 300 are surrounded by sensitive edges SG, which are fixed to the perimeter of the segment 14 as may be seen in Figure 13, i.e., in a position corresponding to the gap between the modules 280, 300 and the segment 14 itself. As is known, the sensitive edges are generally obtained by means of hollow gaskets with specific standardized electrical resistance. In the case where a finger inadvertently happens to be pinched between the segment 14 and one of the moving modules 280, 300, pinching would cause squeezing of the sensitive edge SG, thus creating a short-circuit, which, detected by the control unit of the bed 1, would lead to immediate interruption of the supply to the actuators that govern the modules 280, 300.

For the rest, there applies, where not manifestly incompatible, the same description already developed as regards the elements 28, 30 and 28', 30', in particular as regards operation of the modules 280, 300.

The person skilled in the branch will likewise appreciate that the diffusion modules 28 and 30, 28' and 30', 280 and 300 are suited to installation on objects even markedly different from the bed 1. In particular, they are suited to installation, with similar functions and working modalities, on funiture and fixtures such as, for example, a seat, an armchair, a couch, etc. It is consequently possible to provide one or more of the above furnishings by integrating therein the aforesaid diffusion modules.

## Claims

1. A multi-functional bed (1) including:
- a base (2); and
- a frame (4) supported by said base (2),
wherein:
- said frame (4) is configured for receiving a user (U) and includes an end region (H) wherein, when a user (U) is lying on the bed (1), the head (HE) of the user (U) is received; and
- the bed (1) includes a first and a second diffusion modules (28, 30; 28', 30'; 280, 300), which located in said end region (H) and configured for diffusion of at least one of an active agent, a sound signal, and a visual signal,
each of said diffusion modules (28, 30; 28', 30'; 280, 300) being movable between a resting position and a working position, wherein, in said working position, said diffusion modules (28, 30; 28', 30'; 280, 300) are oriented towards the head (HE) of the user (U),
and further wherein:
- said diffusion modules (28, 30; 28', 30'; 280, 300) are arranged on opposite sides of said end region (H) so that, when a user (U) is lying on the bed (1), the head (HE) of the user (U) is received between the diffusion modules (28, 30; 28', 30'), and
- each of said diffusion modules (28, 30; 28', 30') is rotatable about a respective axis of rotation (X28, X30) between said resting position and said working position, wherein the axes (X28, X30) are coplanar and convergent.

2. The multi-functional bed (1) according to Claim 1, wherein each of said diffusion modules (28, 30; 28', 30'; 280, 300) is installed on a winglet (WL28, WL30), which is rotatable around a respective axis of rotation (X28, X30) between said resting position and said working position, wherein the axes of rotation (X28, X30) of said winglets (WL28, WL30) are convergent to one another.

3. The multi-functional bed (1) according to Claim 1, wherein each diffusion module (28, 30; 28', 30'; 280, 300) includes at least one of a speaker (SP), a diffuser of active agent (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3), and a light sources strip (58; 580).

4. The multi-functional bed (1) according to Claim 3, wherein each diffusion module (28, 30; 28', 30'; 280, 300) includes said speaker (SP), said diffuser of active agent, in particular a fragrance diffuser (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) and said light sources strip (58; 580), said speaker (SP) and said light sources strip (58; 580) being installed on surfaces (54*, 58*) that are not coplanar and are inclined with respect to one another by an angle (α*) of between 5° and 20°, preferably equal to 8°.

5. The multi-functional bed (1) according to Claim 4, wherein said fragrance diffuser (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) includes one or more channels (44A, 44B, 44C; 46A, 46B, 46C; 48A, 48B, 48C; 44A', 44B', 44C', 46A', 46B', 46C', 48A', 48B', 48C', 460A', 460B', 460C'; 440A, 440B, 440C, 460_1, 460_2, 460_3, 480A, 480B, 480C, 4600_1, 4600_2, 4600_3), each communicating with a corresponding fan (52A, 52B, 52C; 520A, 520B, 520C) configured for supplying an air flow therein, wherein in each channel a cartridge (F1, F2, F3; F1L, F2L, F3L) containing one or more fragrances is housed.

6. The multi-functional bed (1) according to Claim 5, wherein each channel is doubled (46A', 46B', 46C', 460A', 460B', 460C'; 460_1, 460_2, 460_3, 4600_1, 4600_2, 4600_3) including a first portion (46A', 46B', 46C'; 460_1, 460_2, 460_3) and a second portion (460A', 460B', 460C'; 4600_1, 4600_2, 4600_3), wherein housed in each first portion (46A', 46B', 46C'; 460_1, 460_2, 460_3) is a cartridge containing a solid state fragrance (F1, F2, F3), whereas housed in each second portion is a vial containing a liquid state fragrance (F1L, F2L, F3L), wherein the two portions of each channel are in communication with one another so as to enable mixing of the solid state and liquid state fragrances entrained by the air flow supplied by each of the fans (52A, 52B, 52C; 520A, 520B, 520C).

7. The multi-functional bed (1) according to Claim 6, wherein said channels are made in a cartridge element (460), associated to which is a radio-frequency transponder (RFT) configured for communication with a radio-frequency reader (RFR) installed in the corresponding diffusion module.

8. The multi-functional bed (1) according to Claim 7, wherein said radio-frequency transponder (RFT) contains information corresponding to at least one of the following:
- a code identifying the origin of the cartridge element (460);
- one or more active agents (F1, F2, F3, F1L, F2L, F3L) contained in said cartridge element (460); and
- an optimal setting of operating parameters of the bed (1) according to characteristics of the one or more active agents contained in said cartridge element (460) .

9. The multi-functional bed (1) according to any one of the preceding claims, wherein said frame includes a plurality of articulated segments (14, 16, 18, 20) that can be displaced to obtain different configurations of said bed (1), said frame (4) including a surface (S) adapted to receive a user (U) and defined by a plurality of plates (S), each fixed to a corresponding articulated segment, wherein each plate (S) carries one or more light sources strips (ST).

10. The multi-functional bed (1) according to Claim 7, moreover including one or more cushions (CSH), preferably with a padding transparent to the light emitted by said light sources strips (ST), laid on the plates (S) that define the surface adapted to receive the user (U), wherein said plates (S) further carry one or more pad-type electrical-resistance heaters for heat-conditioning of said cushions (CSH).

11. The multi-functional bed (1) according to any one of the preceding claims, including a plurality of anti-pinch devices configured for stopping the movement of one or more mobile parts (12A; 14, 16, 18, 20, 280, 300) of the bed with respect to one or more elements of the bed fixed with respect to said one or more mobile parts (12A; 14, 16, 18, 20, 280, 300) during said movement when a foreign body enters the path of said movement.

12. The multi-functional bed (1) according to Claim 9, wherein said plurality of anti-pinch devices includes at least one of:
- a first anti-pinch device (MS1) configured for stopping movement of one or more segments (14, 18, 20) of said frame (4) in the case where a foreign body comes to be present on a supporting element (10*) of said frame (4) in a position underlying said one or more segments (14, 18, 20),
- a second anti-pinch device (MS2) configured for stopping the movement of telescopic uprights (6, 8) of said base (2) that support said frame (4) in the case where a foreign body comes into contact with a shelf structure (12, 12A), which is suspended between said telescopic uprights (6, 8) and is vertically mobile therewith; and
- a third anti-pinch device (SG) configured for stopping movement of said first and/or second diffusion modules (280, 300) in the case where a foreign body comes to be pinched between them and a segment (14) of said frame (4) that receives the head of a user.

13. A diffusion module (28, 30; 28', 30'; 280, 300) for use in a multi-functional bed (1) according to any one of the preceding claims, the diffusion module (28, 30; 28', 30'; 280, 300) being provided with stimulation devices including at least one of the following:
- a speaker (SP);
- a diffuser of active agent (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3); and
- a light sources strip (58; 580).

14. The diffusion module (28, 30; 28', 30'; 280, 300) according to Claim 13, including:
- a diffuser of active agent (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3);
- a speaker(SP); and
- a plurality of light sources (58),
wherein each of said stimulation devices (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3; SP; 58; 580) is installed on an active portion (54*, 58*) of the diffusion module (28, 30; 28', 30'; 280, 300) oriented in such a way that, when the diffusion module is in the working position, the stimulus (SS, AS, VS) emitted thereby impinges in a targeted way on a corresponding receptor of the human body.

15. The diffusion module (280, 300) according to Claim 14, including a cartridge element (460), associated to which is a radio-frequency transponder (RFT) configured for communication with a radio-frequency reader (RFR) installed in the diffusion module itself, wherein said radio-frequency transponder (RFT) contains information corresponding to at least one of:
- a code identifying the origin of the cartridge element (460);
- one or more active agents (F1, F2, F3, F1L, F2L, F3L) contained in said cartridge element (460); and
- an optimal setting of operating parameters of the bed (1) according to characteristics of the one or more active agents contained in said cartridge element (460) .

## Patentansprüche

1. Multifunktionsbett (1), umfassend:
- ein Untergestell (2); und
- einen Rahmen (4), der durch das Untergestell (2) getragen wird,
wobei:
- der Rahmen (4) zur Aufnahme eines Benutzers (U) gestaltet ist und einen Endbereich (H) umfasst,
wobei, wenn ein Benutzer (U) auf dem Bett (1) liegt, der Kopf (HE) des Benutzers (U) aufgenommen wird; und
- das Bett (1) ein erstes und ein zweites Diffusionsmodul (28, 30; 28', 30'; 280, 300) enthält, welches sich in dem Endbereich (H) befindet und zur Diffusion eines aktiven Wirkstoffs, eines Tonsignals und/oder eines visuellen Signals gestaltet ist,
jedes der Diffusionsmodule (28, 30; 28', 30'; 280, 300) zwischen einer Ruheposition und einer Arbeitsposition bewegbar ist, wobei die Diffusionsmodule (28, 30; 28', 30'; 280, 300) in der Arbeitsposition auf den Kopf (HE) des Benutzers (U) zu gerichtet sind,
und des Weiteren, wobei:
- die Diffusionsmodule (28, 30; 28', 30'; 280, 300) auf gegenüberliegenden Seiten des Endbereichs (H) angeordnet sind, so dass, wenn ein Benutzer (U) auf dem Bett (1) liegt, der Kopf (HE) des Benutzers (U) zwischen den Diffusionsmodulen (28, 30; 28', 30') aufgenommen wird, und
- jedes der Diffusionsmodule (28, 30; 28', 30') um eine jeweilige Drehachse (X28, X30) zwischen der Ruheposition und der Arbeitsposition drehbar ist, wobei die Achsen (X28, X30) in derselben Ebene liegend und konvergent sind.

2. Multifunktionsbett (1) nach Anspruch 1, wobei jedes der Diffusionsmodule (28, 30; 28', 30'; 280, 300) auf einer Endscheibe (WL28, WL30) installiert ist, die um eine jeweilige Drehachse (X28, X30) zwischen der Ruheposition und der Arbeitsposition drehbar ist, wobei die Drehachsen (X28, X30) der Endscheiben (WL28, WL30) konvergent zueinander sind.

3. Multifunktionsbett (1) nach Anspruch 1, wobei jedes Diffusionsmodul (28, 30; 28', 30'; 280, 300) einen Lautsprecher (SP), einen Wirkstoffdiffusor (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) und/oder einen Lichtquellenstreifen (58; 580) umfasst.

4. Multifunktionsbett (1) nach Anspruch 3, wobei jedes Diffusionsmodul (28, 30; 28', 30'; 280, 300) den Lautsprecher (SP), den Wirkstoffsprühverteiler, insbesondere einen Duftsprühverteiler (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) und den Lichtquellenstreifen (58; 580) enthält, wobei der Lautsprecher (SP) und der Lichtquellenstreifen (58; 580) auf Flächen (54*, 58*) installiert sind, die nicht in derselben Ebene liegend sind und relativ zueinander um einen Winkel (*α**) von zwischen 5° und 20°, vorzugsweise gleich 8°, geneigt sind.

5. Multifunktionsbett (1) nach Anspruch 4, wobei der Duftsprühverteiler (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) einen oder mehrere Kanäle (44A, 44B, 44C; 46A, 46B, 46C; 48A, 48B, 48C; 44A', 44B', 44C', 46A', 46B', 46C', 48A', 48B', 48C', 460A', 460B', 460C'; 440A, 440B, 440C, 460_1, 460_2, 460_3, 480A, 480B, 480C, 4600_1, 4600_2, 4600_3) enthält, die jeweils in Verbindung stehen mit einem entsprechenden Lüfter (52A, 52B, 52C; 520A, 520B, 520C), der zum Zuführen eines Luftstroms darin gestaltet ist, wobei in jedem Kanal eine, einen oder mehrere Duftstoffe enthaltende, Patrone (F1, F2, F3; F1L, F2L, F3L) aufgenommen ist.

6. Multifunktionsbett (1) nach Anspruch 5, wobei jeder Kanal (46A', 46B', 46C', 460A', 460B', 460C'; 460_1, 460_2, 460_3, 4600_1, 4600_2, 4600_3) zweifach mit einem ersten Abschnitt (46A', 46B', 46C'; 460_1, 460_2, 460_3) und einem zweiten Abschnitt (460A', 460B', 460C'; 4600_1, 4600_2, 4600_3) angelegt ist, wobei in jedem ersten Abschnitt (46A', 46B', 46C'; 460_1, 460_2, 460_3) eine Patrone aufgenommen ist, die einen festen Duftstoff (F1, F2, F3) enthält, wogegen in jedem zweiten Abschnitt ein Glasfläschchen aufgenommen ist, das einen Duftstoff im flüssigen Zustand (F1L, F2L, F3L) enthält, wobei die zwei Abschnitte jedes Kanals miteinander in Verbindung stehen, um eine Mischung des Duftstoffs im festen Zustand und des Duftstoffs im flüssigen Zustand zu ermöglichen, die durch den von jedem der Lüfter (52A, 52B, 52C; 520A, 520B, 520C) zugeführten Luftstrom mitgerissen werden.

7. Multifunktionsbett (1) nach Anspruch 6, wobei die Kanäle in einem Patronenelement (460) gebildet sind, mit dem ein zur Kommunikation mit einem in dem jeweiligen Diffusionsmodul eingebauten Hochfrequenzleser (RFR) ausgeführter Hochfrequenztransponder (RFT) verbunden ist.

8. Multifunktionsbett (1) nach Anspruch 7, wobei der Hochfrequenztransponder (RFT) Informationen enthält, die zumindest einem der folgenden entsprechen:
- einem Code, der den Ursprung des Patronenelements (460) erkennt;
- einem oder mehreren im Patronenelement (460) enthaltenen Wirkstoffen (F1, F2, F3, F1L, F2L, F3L); und
- einer optimalen Einstellung von Betriebsparametern des Bettes (1) entsprechend Eigenschaften des einen oder mehrerer Wirkstoffe, die in dem Patronenelement (460) enthalten sind.

9. Multifunktionsbett (1) nach einem der vorhergehenden Ansprüche, wobei der Rahmen eine Vielzahl von Gelenksegmenten (14, 16, 18, 20) enthält, die verschoben werden können, um unterschiedliche Anordnungen des Bettes (1) zu erhalten, wobei der Rahmen (4) eine Fläche (S) einschließt, die einen Benutzer (U) aufnehmen soll und durch eine Vielzahl von Platten (S) gebildet wird, die jeweils an einem entsprechenden Gelenksegment befestigt sind, wobei jede Platte (S) einen oder mehrere Lichtquellenstreifen (ST) trägt.

10. Multifunktionsbett (1) nach Anspruch 7, das des Weiteren ein oder mehrere Polster (CSH) vorzugsweise mit einer Füllung umfasst, die Licht durchlässt, das von den Lichtquellenstreifen (ST) ausgesendet wird, die auf die Platten (S) gelegt werden, die die Oberfläche bilden und angepasst sind, den Benutzer (U) aufzunehmen, wobei die Platten (S) außerdem eine oder mehrere polsterartige Widerstandsheizelemente zur Wärmebehandlung der Polster (CSH) tragen.

11. Multifunktionsbett (1) nach einem der vorhergehenden Ansprüche, das eine Vielzahl von Vorrichtungen gegen Klemmens umfasst, die ausgeführt sind, die Bewegung eines oder mehrerer beweglicher Teile (12A; 14, 16, 18, 20, 280, 300) des Betts relativ zu einem oder mehreren Elementen des Betts, die bezüglich des einen oder mehrerer mobiler Teile (12A; 14, 16, 18, 20, 280, 300) während der Bewegung festgelegt sind, zu unterbrechen, wenn ein Fremdkörper in den Weg der Bewegung eintritt.

12. Multifunktionsbett (1) nach Anspruch 9, wobei die Vielzahl von Vorrichtungen gegen Klemmens:
- eine erste Vorrichtung gegen Klemmens (MS1), die zur Unterbrechung einer Bewegung eines oder mehrerer Segmente (14, 18, 20) des Rahmens (4) in dem Fall ausgeführt ist, wenn es dazu kommt, dass ein Fremdkörper auf einem Stützelement (10*) des Rahmens (4) in einer Position vorhanden ist, die unter dem einen oder mehreren Segmenten (14, 18, 20) liegt,
- eine zweite Vorrichtung gegen Klemmens (MS2), die ausgeführt ist zur Unterbrechung der Bewegung von teleskopischen Stützen (6, 8) des Untergestells (2), die den Rahmen (4) in dem Fall unterstützen, wenn ein Fremdkörper mit einer Fachbodenstruktur (12, 12A) in Berührung kommt, die zwischen den teleskopischen Stützen (6, 8) aufgehängt ist und mit diesen vertikal beweglich ist; und/oder
- eine dritte Vorrichtung gegen Klemmens (SG) enthält, die zur Unterbrechung einer Bewegung des ersten Diffusionsmoduls und/oder des zweiten Diffusionsmoduls (280, 300) in dem Fall ausgeführt ist, wenn es dazu kommt, dass ein Fremdkörper zwischen ihnen und einem Segment (14) des Rahmens (4), das den Kopf eines Benutzers aufnimmt, eingeklemmt wird.

13. Diffusionsmodul (28, 30; 28', 30'; 280, 300) zur Verwendung in einem Multifunktionsbett (1) nach einem der vorhergehenden Ansprüche, wobei das Diffusionsmodul (28, 30; 28', 30'; 280, 300) mit Stimulationseinrichtungen versehen ist, die:
- einen Lautsprecher (SP);
- einen Wirkstoffdiffusor (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3) und/oder
- einen Lichtquellenstreifen (58; 580) umfassen.

14. Diffusionsmodul (28, 30; 28', 30'; 280, 300) nach Anspruch 13, umfassend:
- einen Wirkstoffdiffusor (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3);
- einen Lautsprecher (SP); und
- eine Vielzahl von Lichtquellen (58),
wobei jede der Stimulationseinrichtungen (52A, 52B, 52C, 44A, 44B, 44C, 46A, 46B, 46C, 48A, 48B, 48C; 44A', 44B', 44C', 46A', 46B', 46C', 48A', 48B', 48C', 460A', 460B', 460C'; 520A, 520B, 520C, 440A, 440B, 440C, 460_1, 460_2, 460_3, 480A, 480B, 480C, 4600_1, 4600_2, 4600_3; SP; 58; 580) an einem aktiven Teil (54*, 58*) des Diffusionsmoduls (28, 30; 28', 30'; 280, 300) derart ausgerichtet installiert ist, dass, wenn sich das Diffusionsmodul in der Arbeitsposition befindet, die dadurch ausgesendete Anregung (SS, AS, VS) in gezielter Weise auf einem entsprechenden Reizempfänger des menschlichen Körpers auftrifft.

15. Diffusionsmodul (280, 300) nach Anspruch 14, umfassend ein Patronenelement (460), mit dem ein zur Kommunikation mit einem in dem Diffusionsmodul selbst eingebauten Hochfrequenzleser (RFR) ausgeführter Hochfrequenztransponder (RFT) verbunden ist, wobei der Hochfrequenztransponder (RFT) Informationen enthält, die zumindest einem der folgenden entsprechen:
- einem Code, der den Ursprung des Patronenelements (460) erkennt;
- einem oder mehreren in dem Patronenelement (460) enthaltenen Wirkstoffen (F1, F2, F3, F1L, F2L, F3L); und
- einer optimalen Einstellung von Betriebsparametern des Bettes (1) entsprechend Eigenschaften des einen oder mehrerer Wirkstoffe, die in dem Patronenelement (460) enthalten sind.

## Revendications

1. Lit multifonctionnel (1) comportant :
- une base (2) ; et
- un châssis (4) supporté par ladite base (2),
dans lequel :
- ledit châssis (4) est configuré pour recevoir un utilisateur (U) et comporte une région d'extrémité (H), dans lequel,
lorsqu'un utilisateur (U) est allongé sur le lit (1), la tête (HE) de l'utilisateur (U) est reçue ; et
- le lit (1) comporte des premier et deuxième modules de diffusion (28, 30 ; 28', 30' ; 280, 300), qui sont situés dans ladite région d'extrémité (H) et configurés pour diffuser au moins l'un parmi un agent actif, un signal sonore, et un signal visuel,
chacun desdits modules de diffusion (28, 30 28', 30' ; 280, 300) étant mobile entre une position de repos et une position de travail, où, dans ladite position de travail, lesdits modules de diffusion (28, 30 ; 28', 30' ; 280, 300) sont orientés vers la tête (HE) de l'utilisateur (U),
et dans lequel en outre :
- lesdits modules de diffusion (28, 30 ; 28', 30' ; 280, 300) sont agencés sur des côtés opposés de ladite région d'extrémité (H) de manière que, lorsqu'un utilisateur (U) repose sur le lit (1), la tête (HE) de l'utilisateur (U) est reçue entre les modules de diffusion (28, 30 ; 28', 30'), et
- chacun desdits modules de diffusion (28, 30 ; 28', 30') peut tourner autour d'un axe de rotation respectif (X28, X30) entre ladite position de repos et ladite position de travail, où les axes (X28, X30) sont coplanaires et convergents.

2. Lit multifonctionnel (1) selon la revendication 1, dans lequel chacun desdits modules de diffusion (28, 30 ; 28', 30' ; 280, 300) est installé sur une ailette (WL28, WL30), qui peut tourner autour d'un axe de rotation respectif (X28, X30) entre ladite position de repos et ladite position de travail, où les axes de rotation (X28, X30) desdites ailettes (WL28, WL30) sont convergents l'un vers l'autre.

3. Lit multifonctionnel (1) selon la revendication 1, dans lequel chaque module de diffusion (28, 30 ; 28', 30' ; 280, 300) comporte au moins l'un parmi un haut-parleur (SP), un diffuseur d'agent actif (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3, 480A, 480B, 480C, 4600_1, 4600_2, 4600_3), et une bande de sources de lumière (58 ; 580).

4. Lit multifonctionnel (1) selon la revendication 3, dans lequel chaque module de diffusion (28, 30 ; 28', 30' ; 280, 300) comporte ledit haut-parleur (SP), ledit diffuseur d'agent actif, en particulier un diffuseur de parfum (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3, 480A, 480B, 480C, 4600_1, 4600_2, 4600_3) et ladite bande de sources de lumière (58 ; 580), ledit haut-parleur (SP) et ladite bande de sources de lumière (58 ; 580) étant installés sur des surfaces (54*, 58*) qui ne sont pas coplanaires et sont inclinées l'une par rapport à l'autre d'un angle (α*) compris entre 5° et 20°, de préférence égal à 8°.

5. Lit multifonctionnel (1) selon la revendication 4, dans lequel ledit diffuseur de parfum (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3, 480A, 480B, 480C, 4600_1, 4600_2, 4600_3) comporte un canal ou plusieurs canaux (44A, 44B, 44C ; 46A, 46B, 46C ; 48A, 48B, 48C ; 44A', 44B', 44C', 46A', 46B', 46C', 48A', 48B', 48C', 460A', 460B', 460C' ; 440A, 440B, 440C, 460_1, 460_2, 460_3, 480A, 480B, 480C, 4600_1, 4600_2, 4600_3), chacun communiquant avec un ventilateur correspondant (52A, 52B, 52C ; 520A, 520B, 520C) configuré pour fournir un flux d'air à l'intérieur de celui-ci, où dans chaque canal, une cartouche (F1, F2, F3 ; F1L, F2L, F3L) contenant un ou plusieurs parfum(s) est logée.

6. Lit multifonctionnel (1) selon la revendication 5, dans lequel chaque canal est doublé (46A', 46B', 46C', 460A', 460B', 460C' ; 460_1, 460_2, 460_3, 4600_1, 4600_2, 4600_3) comportant une première partie (46A', 46B', 46C' ; 460_1, 460_2, 460_3) et une deuxième partie (460A', 460B', 460C' ; 4600_1, 4600_2, 4600_3), où dans chaque première partie (46A', 46B', 46C' ; 460_1, 460_2, 460_3) est logée une cartouche contenant un parfum à l'état solide (F1, F2, F3), alors que dans chaque deuxième partie se trouve un flacon contenant un parfum à l'état liquide (F1L, F2L, F3L), où les deux parties de chaque canal sont en communication l'une avec l'autre de manière à permettre le mélange des parfums à l'état solide et à l'état liquide entraînés par le flux d'air fourni par chacun des ventilateurs (52A, 52B, 52C ; 520A, 520B, 520C).

7. Lit multifonctionnel (1) selon la revendication 6, dans lequel lesdits canaux sont réalisés dans un élément de cartouche (460), auquel est associé un transpondeur radiofréquence (RFT) configuré pour communiquer avec un lecteur radiofréquence (RFR) installé dans le module de diffusion correspondant.

8. Lit multifonctionnel (1) selon la revendication 7, dans lequel ledit transpondeur radiofréquence (RFT) contient des informations correspondant à au moins l'un des éléments suivants :
- un code identifiant l'origine de l'élément de cartouche (460) ;
- un ou plusieurs agent(s) actif(s) (F1, F2, F3, F1L, F2L, F3L) contenu(s) dans ledit élément de cartouche (460) ; et
- un réglage optimal des paramètres de fonctionnement du lit (1) selon des caractéristiques du ou des plusieurs agent(s) actif(s) supplémentaire(s) contenu(s) dans ledit élément de cartouche (460).

9. Lit multifonctionnel (1) selon l'une quelconque des revendications précédentes, dans lequel ledit châssis comporte une pluralité de segments articulés (14, 16, 18, 20) qui peuvent être déplacés pour obtenir différentes configurations dudit lit (1), ledit châssis (4) comportant une surface (S) adaptée pour recevoir un utilisateur (U) et définie par une pluralité de plaques (S), chacune étant fixée à un segment articulé correspondant, où chaque plaque (S) porte une ou plusieurs bande(s) de sources de lumière (ST).

10. Lit multifonctionnel (1) selon la revendication 7, comportant en outre un ou plusieurs coussin(s) (CSH), ayant de préférence un rembourrage transparent à la lumière émise par lesdites bandes de sources de lumière (ST), posés sur les plaques (S) qui définissent la surface adaptée pour recevoir l'utilisateur (U), où lesdites plaques (S) portent en outre un ou plusieurs élément(s) chauffant(s) à résistance électrique de type à rembourrage pour le conditionnement thermique desdits coussins (CSH).

11. Lit multifonctionnel (1) selon l'une quelconque des revendications précédentes, comportant une pluralité de dispositifs anti-coincement configurés pour arrêter le mouvement d'une ou de plusieurs partie(s) mobile(s) (12A ; 14, 16, 18, 20, 280, 300) du lit avec par rapport à un ou plusieurs élément(s) du lit fixé(s) par rapport à ladite/auxdites une ou plusieurs partie(s) mobile(s) (12A ; 14, 16, 18, 20, 280, 300) pendant ledit mouvement lorsqu'un corps étranger pénètre dans le trajet dudit mouvement.

12. Lit multifonctionnel (1) selon la revendication 9, dans lequel ladite pluralité de dispositifs anti- coincement comporte au moins un des éléments suivants :
- un premier dispositif anti- coincement (MS1) configuré pour arrêter le mouvement d'un ou de plusieurs segment(s) (14, 18, 20) dudit châssis (4) dans le cas où un corps étranger vient à être présent sur un élément de support (10*) dudit châssis (4) dans une position sous-jacente audit un ou plusieurs segment(s) (14, 18, 20),
- un deuxième dispositif anti-coincement (MS2) configuré pour arrêter le mouvement de montants télescopiques (6, 8) de ladite base (2) qui supportent ledit châssis (4) dans le cas où un corps étranger vient en contact avec une structure d'étagère (12, 12A), qui est suspendue entre lesdits montants télescopiques (6, 8) et est mobile verticalement avec ceux-ci ; et
- un troisième dispositif anti-coincement (SG) configuré pour arrêter le mouvement dudit/desdits premier et/ou deuxième module(s) de diffusion (280, 300) dans le cas où un corps étranger vient à être coincé entre eux et un segment (14) dudit un châssis (4) qui reçoit la tête d'un utilisateur.

13. Module de diffusion (28, 30 28', 30' ; 280, 300) pour une utilisation dans un lit multifonctionnel (1) selon l'une quelconque des revendications précédentes, le module de diffusion (28, 30 ; 28', 30' ; 280, 300) étant muni de dispositifs de stimulation comportant au moins l'un des éléments suivants :
- un haut-parleur (SP) ;
- un diffuseur d'agent actif (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3, 480A, 480B, 480C, 4600_1, 4600_2, 4600_3) ; et
- une bande de sources de lumière (58 ; 580).

14. Module de diffusion (28, 30; 28', 30' 280, 300) selon la revendication 13, comportant :
- un diffuseur d'agent actif (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3 , 480A, 480B, 480C, 4600_1, 4600_2, 4600_3) ;
- un haut-parleur (SP) ; et
- une pluralité de sources de lumière (58),
dans lequel chacun desdits dispositifs de stimulation (52A, 52B, 52C, 44A, 44B, 44C, 46A, 46B, 46C, 48A, 48B, 48C ; 44A', 44B', 44C', 46A', 46B', 46C', 48A', 48B', 48C', 460A', 460B', 460C' ; 520A, 520B, 520C, 440A, 440B, 440C, 460_1, 460_2, 460_3, 480A, 480B, 480C, 4600_1, 4600_2, 4600_3 ; SP ; 58 ; 580) est installé sur une partie active (54*, 58*) du module de diffusion (28, 30 ; 28', 30' ; 280, 300) orientée de manière que, lorsque le module de diffusion est en position de travail, le stimulus (SS, AS, VS) émis par celui-ci arrive, de manière ciblée, sur un récepteur correspondant du corps humain.

15. Module de diffusion (280, 300) selon la revendication 14, comportant un élément de cartouche (460), auquel est associé un transpondeur radiofréquence (RFT) configuré pour communiquer avec un lecteur radiofréquence (RFR) installé dans le module de diffusion lui-même, dans lequel ledit transpondeur radiofréquence (RFT) contient des informations correspondant à au moins l'un des éléments suivants :
- un code identifiant l'origine de l'élément de cartouche (460) ;
- un ou plusieurs agent(s) actif(s) (F1, F2, F3, F1L, F2L, F3L) contenu(s) dans ledit élément de cartouche (460) ; et
- un réglage optimal des paramètres de fonctionnement du lit (1) selon des caractéristiques du ou des plusieurs agent(s) actif(s) supplémentaire(s) contenu(s) dans ledit élément de cartouche (460).
